# EUROPEAN PATENT SPECIFICATION

(11) **EP 2 257 814 B1**
(45) Date of publication and mention of the grant of the patent: **29.08.2012**
(21) Application number: 09721964.6
(22) Date of filing: 17.03.2009
(51) Int. Cl.: G01N 33/68

(54) **DIAGNOSTIC OF PRE-SYMPTOMATIC METABOLIC SYNDROME**
DIAGNOSE DES PRÄSYMPTOMATISCHEN STOFFWECHSELSYNDROMS
DIAGNOSTIC DU SYNDROME MÉTABOLIQUE PRÉSYMPTOMATIQUE

(30) Priority: 21.03.2008 US 38433; 15.05.2008 EP 08156294
(43) Date of publication of application: 08.12.2010
(73) Proprietor: Podiceps B.v., 3994 EJ Houten (NL)
(72) Inventor: DE WIT, Nicole Johanna Wilhelmina, NL-5476 LS Vorstenbosch (NL); VAN DER MEER, Roelof, NL-6712 HA Ede (NL)
(74) Representative: Swinkels, Bart Willem
(86) International application number: PCT/NL2009/050126
(87) International publication number: WO 2009/116861

(56) References cited:
- EP-A- 1 398 032
- WO-A-2007/047796
- FR-A- 2 893 136

## Description

### Field of the invention

The invention relates to a method for diagnosing pre-symptomatic metabolic syndrome in a subject, wherein said method comprises determining the expression level of a gene represented by a nucleotide sequence selected from the group consisting of SEQ ID NO:1-18 in a subject.

### Background of the invention

Metabolic syndrome is a multi-component condition associated with a high risk of type 2 diabetes mellitus and cardiovascular disease (38) and the onset of cancer. In the industrialized societies, approximately 20-40% of the population are affected by the metabolic syndrome and its incidence is expected to rise even further in the next decades (31). Obesity and insulin resistance are two major risk factors underlying the metabolic syndrome. Obesity is considered the principal instigator that predisposes to insulin resistance, which is the pivotal metabolic disturbance in the metabolic syndrome (25).

Lifestyle factors, such as nutrition and limited physical activity, are known to contribute to the pathogenesis of obesity and insulin resistance. The association between development of these disorders and excessive intake of dietary fat is frequently studied, especially in obesity-prone C57BL/6J mice (2, 27, 34, 43, 51). Most of these studies are focused on the physiology and underlying molecular mechanisms in liver, skeletal muscle and adipose tissue, as these organs are target organs of insulin-modulated metabolism (6, 30, 46). However, there is growing evidence that also the small intestine can play an essential role in the etiology of obesity and/or insulin resistance, due to its gatekeeper function at the physical interphase between body and diet. Next to an efficient uptake of nutrients, the enterocytes in the small intestine are also responsible for sensing of luminal contents that are modulated by the diet. As a result of this sensing, the small intestine secretes signaling molecules, such as gut hormones and pro- and anti-inflammatory cytokines, to which liver, muscle and adipose tissue can respond by modulating their metabolism to keep homeostatic control. Potential small intestinal factors that contribute to development of metabolic syndrome are specific effects of gut hormones on satiety and glucose homeostasis (9, 12), diminished fatty acid oxidative capacity of enterocytes (27) and gut microbiota composition (2, 48). FR2893136A relates to a marker for hepatic diseases.

Due to the growing importance of metabolic syndrome in western societies, there is a great need for specific markers that could be used in a method for diagnosing pre-symptomatic metabolic syndrome in a subject. Such markers are not available yet.

### Description of the invention

In this inventory study, we investigated the potential role of the small intestine in development of dietary fat-induced obesity and/or insulin resistance in C57BL/6J mice in a rather comprehensive way during time. Therefore, we performed microarray analysis of small intestinal mucosa to explore fat-modulated biological processes and an additional 'secretome' analysis to identify secreted proteins that are able to induce systemic effects underlying the etiology of the metabolic syndrome. Surprisingly, we found that 15 genes among other a Fam3D and/or a ApoA4 gene could be used as specific markers in a method for diagnosing pre-symptomatic metabolic syndrome in a subject.

### Diagnostic method

In a first aspect, there is provided a method for diagnosing pre-symptomatic metabolic syndrome in a subject, the method comprising the steps of:
(a) determining the expression level of a gene represented by a nucleotide sequence selected from the group consisting of SEQ ID NO:1-18 in a subject; and,
(b) comparing the expression level of said gene as defined in (a) with a reference value for said expression level, the reference value preferably being the average value for said expression level in a control subject.

In the context of the invention, metabolic syndrome may be defined as being a multi-component condition associated with a high risk of type 2 diabetes mellitus and cardiovascular disease. Symptomatic metabolic syndrome is generally characterized by at least one of obesity, insulin resistance, type 2 diabetes mellitus and a (cardio)vascular disease. Several methods are already known to diagnose metabolic syndrome (Grundy et al (2004) Circulation, 109: 433-438 and Grundy et al (2005) Circulation, 112: 2735-2752) . Each of these methods define a combination of parameters, for which a specific value or range for each of the parameters will establish the diagnosis of metabolic syndrome in a subject. For example the National Cholesterol Education Program's Adult Treatment Panel III report (so-called ATP III) defines that when at least three of the following parameters being body weight, lipid concentration, blood pressure, glucose are comprised within a specific range as defined in table 1 of Grundy et al (2004) or in table 1 of Grundy et al (2005), metabolic syndrome will be diagnosed. As another example, the World Health Organization (WHO) proposed another definition wherein the presence of insulin resistance, in combination with two of the following parameters being body weight, lipid, blood pressure and glucose being comprised within a specific range as defined in table 2 of Grundy et al (2004) or table 1 of Grundy et al (2005) metabolic syndrome is diagnosed. Using any of the existing methods (such as ATP III or WHO definitions) for diagnosing metabolic syndrome leads to a relative late diagnosis of the syndrome, which means that the course of the syndrome is quite difficult to be reversed in a subject.

In the context of the invention, diagnosing pre-symptomatic metabolic syndrome preferably means that a diagnosis is reached before the actual development of a symptomatic metabolic syndrome as earlier defined herein. The invention allows a specific and early detection of metabolic syndrome, which will allow to reverse the course of the syndrome more easily in a subject. In addition, the target genes or proteins identified in the invention may be effected by other means to reverse or stop the development of metabolic syndrome and the related diseases. The invention is the first known to allow a detection of a pre-symptomatic metabolic syndrome. A detection of a pre-symptomatic metabolic syndrome is preferably reached earlier in time than the detection of symptomatic metabolic syndrome using any of the other methods (or definitions) earlier defined herein. In this context, "earlier in time" preferably means at least one day, at least two days, at least three days, at least four days, at least five days, at least six days, at least seven days, at least eight days, at least nine days, at least ten days at least 15 days, at least 20 days, at least 25 days, at least 30 days, at least 1 month, at least 2 months, at least 3 months, at least 4 months, at least 5 months, at least 6 months, at least 7 months, at least 8 months, at least 9 months, at least 10 months, at least 11 months, at least 12 months or more before the actual development of a symptomatic metabolic syndrome.

In the context of the invention, diagnosis preferably means a predictive risk assessment of the subsequent development of metabolic syndrome in a subject.

In the context of the invention, a subject may be an animal or a human being. In principle, any subject could be diagnosed using the method of the invention. The diagnosis method may be applied as often as necessary in a subject. Preferably, a subject diagnosed is a subject suspected to have a high risk of developing a metabolic syndrome, due for example to potential genetic predisposition, and/or to the age of the subject and/or to the lifestyle of a subject (for example nutritional habit and/or to the absence of physical activity). Preferably, a subject is a human being.

In the context of the invention, "a gene or nucleotide molecule as identified herein" preferably means a gene or nucleotide molecule represented by a nucleotide sequence selected from the group consisting of SEQ ID NO:1-18, more preferably from the group consisting of SEQ ID NO:1, 2, 3, 4, 5, 6, 7, 8, 9, 10, 11 or 12, 13, 14, 15 or 16 or 17 and 18. Even more preferably, the group consists of SEQ ID NO:1 and 2.

In the context of the invention, "a polypeptide or protein as identified herein" preferably means a polypeptide encoded by a gene or nucleotide molecule as identified herein.

In the context of the invention, "a reference value" for the expression level of a gene as identified herein is preferably the average value for said expression level in control subjects. More preferably, a control subject is a subject, who has not developed a metabolic syndrome as diagnosed by any of the methods as mentioned earlier herein. Alternatively according to an even more preferred embodiment, a control subject is a subject who has not developed any of the characteristics (i.e. parameters) of the metabolic syndrome yet. For example, a subject will not be said to have abdominal obesity, lipid, blood pressure, glucose and/or insulin resistance as defined in table 1 of Grundy et al (2005).

The assessment of the expression level of a gene as identified herein may be realised at the protein expression level (quantifying the amount of a protein encoded by said genes as identified herein), and/or by quantifying the amount of a gene (or nucleotide molecule) encoding said protein (both the reference value from a control subject and the value from a subject wherein the method is being carried out). Table 5 (and genes marked in grey in table 4) identifies 15 genes represented by 18 nucleotide sequences SEQ ID NO:1-18 and corresponding encoded polypeptides or proteins. Each of these genes can be used alone or in combination or in sub-combinations as a marker for pre-symptomatic metabolic syndrome. They were all found up-regulated in the studied animal model, their expression product is secreted and detectable in blood and their expression is restricted to a limited number of tissues. Each of these features renders these genes attractive to be used as a marker for diagnosing pre-symptomatic metabolic syndrome and as target for interfering in the development of full blown metabolic syndrome and consequentially the related diseases. The invention encompasses the use of 1, 2, 3, 4, 5, 6, 7, 8, 9, 10, 11, 12, 13, 14 till 15 genes represented by SEQ ID NO:1-18. It is to be noted that both genes are represented by more than one nucleotide sequences: the Pap gene is represented by two nucleotides sequences SEQ ID NO:11 and 12, the Reg3g gene by three SEQ ID NO:15, 16 and 17. These two (respectively three) nucleotide sequences code for one polypeptide represented by the same amino acid sequence SEQ ID NO:29 (respectively SEQ ID NO:32). Therefore, when referring to the Pap (respectively the Reg3g) gene, one may use either of the identified nucleotide sequences. Fam3D (Oit1, represented by SEQ ID NO:1) and ApoA4 (represented by SEQ ID NO:2) are gut-specific markers (small intestine), their differences in gene expression as measured in serum may easily be extrapolated to differences in gene expression in the small intestine. Therefore, the use of these genes represented by SEQ ID NO:1 and/or SEQ ID NO:2 is preferred in a diagnostic method for pre-symptomatic metabolic syndrome.

The skilled person will understand that for each identified gene (or nucleotide sequence) and corresponding polypeptide or protein, it is possible to isolate multiple isoforms of a given protein depending on the subject to be tested. It is to be understood that each gene as identified herein by a given Sequence Identity Number (SEQ ID NO) is not limited to this specific sequence. Each gene sequence or nucleotide sequence as identified herein encodes a given protein or polypeptide as identified in table 5. Throughout this application, each time one refers to a specific nucleotide sequence SEQ ID NO (take SEQ ID NO:1 as example), one may replace it by:
i. a polypeptide comprising an amino acid sequence that has at least 60% sequence identity with amino acid sequence SEQ ID NO:19 (as identified in table 5) as being encoded by SEQ ID NO:1,
ii.a nucleotide sequence comprising a nucleotide sequence that has at least 60% sequence identity with SEQ ID NO:1 (as example).
iii. a nucleotide sequence the complementary strand of which hybridizes to a nucleotide sequence of (ii);
iv. a nucleotide sequence the sequence of which differs from the sequence of a nucleotide sequence of (iii) due to the degeneracy of the genetic code.
iv. a nucleotide sequence that encodes an amino acid sequence that has at least 60% amino acid identity with an amino acid sequence encoded by a nucleotide sequence SEQ ID NO:1.

Each nucleotide sequence or amino acid sequence described herein by virtue of its identity percentage (at least 60%) with a given nucleotide sequence or amino acid sequence respectively has in a further preferred embodiment an identity of at least 65%, 70%, 75%, 80%, 85%, 90%, 95%, 97%, 98%, 99% or more identity with the given nucleotide or amino acid sequence respectively. In a preferred embodiment, sequence identity is determined by comparing the whole length of the sequences as identified herein.

Identity is later herein defined. The quantification of the amount of a gene (or nucleotide molecule) as identified herein is preferably performed using classical molecular biology techniques such as (real time) PCR, arrays or northern analysis. In this embodiment, a gene (or nucleotide molecule) encoding a polypeptide as defined herein means a messenger RNA (mRNA). Alternatively, according to another preferred embodiment, in the diagnosis method the expression level of a polypeptide is determined directly by quantifying the amount of said polypeptide. Quantifying a polypeptide amount may be carried out by any known technique. Preferably, a polypeptide amount is quantified using a molecule which specifically binds to said polypeptide. Preferred binding molecules are selected from: an antibody, which has been specifically raised for recognizing a given polypeptide, any other molecule which is known to specifically bind said polypeptide. Such antibody could be used in any immunoassay known to the skilled person such as western blotting, or ELISA (Enzyme-Linked Immuno Sorbent Assay) or FACS (Fluorescence Activated Cell Sorting) using latex beads. The preparation of an antibody is known to those skilled in the art. A short explanation of methods that could be used to prepare antibodies is later herein given. An example of a suitable specific antibody raised against Fam3D is described in US2005/0158753. In the context of the invention, any other molecule known to bind a given polypeptide may be a nucleic acid, e.g. a DNA regulatory region, a polypeptide, a metabolite, a substrate, a regulatory element, a structural component, a chaperone (transport) molecule, a peptide mimetic, a non-peptide mimetic, or any other type of ligand. Mimetic is later herein defined. Binding of a given polypeptide to a second binding molecule may be detected by any standard methods known to those skilled in the art. Suitable methods include affinity chromatography co-electrophoresis (ACE) assays and ELISA. The skilled person will understand that alternatively or in combination with the quantification of a gene encoding a given polypeptide and/or the corresponding polypeptide, the quantification of a substrate of the corresponding polypeptide or of any compound known to be associated with the function of the corresponding polypeptide or the quantification of the function or activity of the corresponding polypeptide using a specific assay is encompassed within the scope of the diagnosis method of the invention. For example, transactivation of a target gene by Fam3D or a Fam3D binding molecule can be determined and quantified, e.g., in a transient transfection assay in which the promoter of the target gene is linked to a reporter gene, e.g., P-galactosidase or luciferase.

Such evaluations can be done *in vitro* or *in vivo* or *ex vivo.*

Since the expression level of a gene (or nucleotide molecule) encoding a polypeptide as identified herein and/or amounts of corresponding polypeptide may be difficult to detect in a subject, a sample from a subject is preferably used. According to another preferred embodiment, the expression level (of a gene or nucleotide molecule or polypeptide) is determined *ex vivo* in a sample obtained from a subject. A sample preferably comprises or consists of a solid of a semi solid sample. Preferred solid or semi solid samples include a part of the small intestine of a subject, also called an intestinal biopsy. Alternatively, a sample preferably comprises or consists of a fluid obtained from a subject. More preferably, a fluid comprises or consists of or is selected from: urine, faeces, blood or saliva. Even more preferably, a fluid is blood plasma. Subsequently, a nucleotide molecule encoding a polypeptide as identified herein and/or said polypeptide are extracted and optionally purified using known methods to the skilled person.

In a more preferred diagnosis method, pre-symptomatic metabolic syndrome is diagnosed when the comparison leads to the finding of a detectable expression of a (i.e. at least one) gene (or nucleotide molecule) and/or of a corresponding polypeptide as identified herein. Alternatively or in combination with earlier preferred embodiment, the comparison leads to the finding of an increase of the expression level of a (i.e. at least one) gene (or nucleotide molecule) and/or of a corresponding polypeptide as identified herein. In control subjects as defmed before, the expression of said gene (or nucleotide molecule) and/or corresponding polypeptide is preferably significantly lower than in subjects diagnosed as having a pre-symptomatic metabolic syndrome.

Detection or an increase of the expression level of a polypeptide as identified herein and/or an increase or a detection of the expression level of a gene (or nucleotide molecule) encoding said polypeptide (or steady state level of said polypeptide) is preferably defined as being a detectable change of the expression level of said polypeptide and/or of a nucleotide molecule encoding said polypeptide (or steady state level of the encoded polypeptide or any detectable change in the biological activity of a polypeptide as defined herein) using a method as defined earlier on as compared to the expression level of a polypeptide as identified herein and/or of a corresponding gene (or nucleotide molecule) (or steady state level of the corresponding encoded polypeptide) in a control subject. According to a preferred embodiment, detection or an increase of the expression level of a gene (or nucleotide molecule) as identified herein is quantified using a specific mRNA assay for the gene (or nucleotide molecule) as earlier defined herein. Preferably, an increase of the expression level of a gene (or nucleotide molecule) encoding a polypeptide as identified herein means an increase of at least 5% of the expression level of the gene (or nucleotide molecule) using PCR. For example, preferred primers used for the PCR for the detection of the expression of a Fam3D gene are identified as SEQ ID NO:34 5'-CTGCCCAGCCAACTACTTTG-3' and SEQ ID NO:35 5'- CTCCCGTGGTTCCATTCAC-3'. More preferably, an increase of the expression level of a gene (or nucleotide molecule) means an increase of at least 10%, even more preferably at least 20%, at least 30%, at least 40%, at least 50%, at least 70%, at least 90%, at least 150% or more. Preferred primers for the PCR for the detection of the expression of a ApoA4 gene are identified as SEQ ID NO:36 5'-CCAAGATCGACCAGAACGTGG -3' a n d SEQ ID N O :37 5'-GTCCTGAGCATAGGGAGCCA -3'.

Preferably, an increase of the expression level of a polypeptide as identified herein means an increase of at least 5% of the expression level of said polypeptide using western blotting and/or using ELISA or a suitable assay. More preferably, an increase of the expression level of a polypeptide means an increase of at least 10%, even more preferably at least 20%, at least 30%, at least 40%, at least 50%, at least 70%, at least 90%, at least 150% or more.

Preferably, an increase of an activity of a given polypeptide as identified herein means an increase of at least 5% of the polypeptide activity using a suitable assay. More preferably, an increase of a polypeptide activity means an increase of at least 10%, even more preferably at least 20%, at least 30%, at least 40%, at least 50%, at least 70%, at least 90%, at least 150% or more.

In a most preferred diagnosis method, pre-symptomatic metabolic syndrome is diagnosed when the comparison leads to the finding of a detectable expression of a Fam3D and/or ApoA4 using PCR and/or an increase of the expression level of a Fam3D and/or ApoA4, said detection or increase being detected at the level of a gene (or nucleotide molecule) encoding a Fam3D (respectively ApoA4) (mRNA) (an increase of at least 5% of the expression level of the gene or nucleotide molecule) using PCR primers as defmed herein.

### Assay device

In a second aspect, the application relates to an assay device for diagnosing pre-symptomatic metabolic syndrome in a subject, wherein the device comprises a molecule which specifically binds a polypeptide as defined earlier herein. More preferably, a device comprises a molecule which specifically binds a Fam3D polypeptide and/or a molecule which specifically binds a ApoA4 polypeptide.

This device may be used in a diagnosis method of the invention. Any subject or physician could use this device at office/home, repeat the use of such device as often as necessary.

The type of molecules that are known to specifically bind a polypeptide as defined herein have already been earlier described herein. In a preferred embodiment, the molecule which specifically binds a polypeptide as identified herein and which is present in the device is an antibody.

In a preferred embodiment, an assay device is a lateral flow test strip also known as dipstick, preferably, though not necessarily, encased in a housing, designed to be read by the subject, and the assay is a sandwich immunoassay. Such devices are impregnated with reagents that specifically indicate the presence of a polypeptide as identified herein by changing colour upon contact with a sample. Preferred subject's samples have already been defined herein. The antibody is preferably labelled by conjugation to a physically detectable label, and upon contacting with the sample containing a polypeptide as identified herein forms a complex. The complex is then contacted with a second antibody, which recognizes the first antibody and which is immobilized on a solid support within the device. The second antibody captures the complex to form a sandwich complex, and the resulting sandwich complex, which is immobilized on the solid support, is detectable by virtue of the label. The test strip may then be inserted into a reader, where the signal from the label in the complex is measured. Alternatively, the test strip could be inserted into the reader prior to addition of the sample. Alternatively and according to a preferred embodiment, the presence of a polypeptide as identified herein is visualised by a subject as a change of colour of at least part of the device. Dipsticks are usually made of paper or cardboard. Usually additional molecules are present in said device as positive or negative controls. A typical positive control could be an antibody recognizing a molecule which is known to be present in a sample to be tested. A typical negative control could be an antibody recognizing a molecule which is known to be absent in a sample to be tested. Accordingly in a further aspect, there is provided the use of such assay device for diagnosing pre-symptomatic metabolic syndrome in a subject, wherein the device comprises a molecule which specifically binds a polypeptide as defined earlier herein. More preferably, a device comprises a molecule which specifically binds a Fam3D polypeptide and/or a molecule which specifically binds a ApoA4 polypeptide. A preferred molecule which specifically binds a Fam3D, respectively a ApoA4 polypeptide is an antibody, more preferably a monoclonal antibody. In another preferred embodiment, such assay is used in a method for diagnosing pre-symptomatic metabolic syndrome as identified herein.

### Method for identification

In a further aspect, there is provided a method for identification of a substance capable of preventing, treating and/or delaying the progression of metabolic syndrome in a subject, the method comprising the steps of:
(a) providing a test cell population or a test animal capable of expressing a gene (or nucleotide molecule) encoding a polypeptide as identified herein and/or a gene (or nucleotide molecule) encoding said polypeptide;
(b) contacting the test cell population or the test animal with a substance;
(c) determining the expression level of a gene (or nucleotide molecule) encoding said polypeptide or the activity or steady state level of said polypeptide in a test cell population or in the test animal contacted with the substance;
(d) comparing the expression, activity or steady state level determined in (c) with the expression, activity or steady state level of the gene (or nucleotide molecule) or of the polypeptide in a test cell population or in a test animal that is not contacted with the substance; and,
(e) identifying a substance that produces a difference in expression level, activity or steady state level of the gene (or nucleotide molecule) or the polypeptide, between the test cell population or test animal that is contacted with the substance and the test cell population or test animal that is not contacted with the substance.

In a preferred embodiment, a test animal is a mouse, more preferably a C57BL/6J mouse. A preferred test cell population comprises mammalian cells, more preferably human cells. Even more preferred cells are colon carcinoma cell lines LS174T and LOVO, since they both express Fam3D.

Alternatively or in combination with previous preferred embodiment in a further preferred embodiment, in step (a), a test cell or a test animal has been modified to over-express a polypeptide as identified herein. This is preferably carried out by transforming a test cell with a nucleic acid construct comprising a nucleotide sequence encoding said polypeptide as defined herein. Alternatively, this is preferably carried out by generating a test animal being transgenic for a given polypeptide as identified herein and as later explained herein. In a further aspect, the invention relates to such a nucleic acid construct. Preferably, a nucleotide sequence is operably linked to a promoter that is capable of driving expression of a nucleotide sequence in a chosen test cell. In a preferred embodiment a nucleic acid construct is a viral gene therapy vector selected from gene therapy vectors based on an adenovirus, an adeno-associated virus (AAV), a herpes virus, a pox virus and a retrovirus. A preferred viral gene therapy vector is an AAV or Lentiviral vector. Such vectors are further described herein below.

Depending on the system used (test cell or test animal), the skilled person will know which conditions are preferred for the contacting step (b).

In step (c), the expression level of a gene (or nucleotide molecule) encoding a polypeptide as identified herein or the activity or steady state level of said polypeptide may be carried out as earlier herein defined.

In a preferred method in step (e), the difference identified in step (d) produced by the substance is a decrease of the expression level of said corresponding gene (or nucleotide molecule), or of the activity or steady state level of said polypeptide.

A decrease of the expression level of a gene (or nucleotide molecule) encoding a polypeptide as identified herein (or steady state level of said polypeptide) is preferably defined as being a detectable change of the expression level of said polypeptide and/or of a gene (or nucleotide molecule) encoding said polypeptide (or steady state level of the encoded polypeptide) or any detectable change in a biological activity of said polypeptide using a method as defined earlier on as compared to the expression level of a given polypeptide and/or of a corresponding gene (or nucleotide molecule) (or steady state level of the corresponding encoded polypeptide in a control subject. According to a preferred embodiment, a decrease of the expression level of a gene (or nucleotide molecule) encoding a given polypeptide as identified herein is quantified using a specific mRNA assay for corresponding gene as earlier defined herein. Preferably, a decrease of the expression level of a gene (or nucleotide molecule) encoding a given polypeptide means a decrease of at least 5% of the expression level of the gene (or nucleotide molecule) using PCR. Preferred primers used for the PCR are already identified herein. More preferably, a decrease of the expression level of a gene (or nucleotide molecule) means a decrease of at least 10%, even more preferably at least 20%, at least 30%, at least 40%, at least 50%, at least 70%, at least 90%, at least 150% or more.

Preferably, a decrease of the expression level of a given polypeptide means a decrease of at least 5% of the expression level of said polypeptide using western blotting and/or using ELISA or a suitable assay. More preferably, a decrease of the expression level of a polypeptide means a decrease of at least 10%, even more preferably at least 20%, at least 30%, at least 40%, at least 50%, at least 70%, at least 90%, at least 150% or more.

Preferably, a decrease of an activity of a given polypeptide means a decrease of at least 5% of the polypeptide activity using a suitable assay. More preferably, a decrease of the polypeptide activity means a decrease of at least 10%, even more preferably at least 20%, at least 30%, at least 40%, at least 50%, at least 70%, at least 90%, at least 150% or more.

In a most preferred method for identifying a substance capable of preventing, treating and/or delaying the progression of metabolic syndrome in a subject when the comparison leads to the comparison leads to a decrease of the expression level of a gene (or nucleotide molecule) as identified herein, said decrease being detected at the level of a gene (or nucleotide molecule) (a decrease of at least 5% of the expression level of the gene (or nucleotide molecule)) using PCR primers as defined herein.

Preferred genes and corresponding polypeptides have already been defined herein.

In one further aspect, the invention also pertains to a substance that is identified in a method the aforementioned methods.

### Sequence identity

"Sequence identity" is herein defined as a relationship between two or more amino acid (polypeptide or protein) sequences or two or more nucleic acid (polynucleotide) sequences, as determined by comparing the sequences. The whole SEQ ID NO may be used or part thereof. In a preferred embodiment, the whole SEQ ID NO as identified herein is used. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg, Gln or Glu; Met to Leu or Ile; Phe to Met, Leu or Tyr; Ser to Thr; Thr to Ser; Trp to Tyr; Tyr to Trp or Phe; and Val to Ile or Leu.

### Antibodies

Some aspects of the invention concern the use of an antibody or antibody-fragment that specifically binds to a polypeptide as identified herein. Methods for generating antibodies or antibody-fragments that specifically bind to a polypeptide are described in e.g. Harlow and Lane (1988, Antibodies: A Laboratory Manual, Cold Spring Harbor Laboratory Press, Cold Spring Harbor, NY) and WO 91/19818; WO 91/18989; WO 92/01047; WO 92/06204; WO 92/18619; and US 6,420,113 and references cited therein. The term "specif binding," as used herein, includes both low and high affinity specific binding. Specific binding can be exhibited, e.g., by a low affinity antibody or antibody-fragment having a Kd of at least about 10⁻⁴ M. Specific binding also can be exhibited by a high affinity antibody or antibody-fragment, for example, an antibody or antibody-fragment having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater.

### Recombinant techniques and methods for over-expression of a polypeptide as identified herein in a test cell or in a test animal

A polypeptide for use in the present invention can be prepared using recombinant techniques, in which a gene (or nucleotide molecule) encoding said polypeptide of interest is (over)expressed in a suitable host cell. The present invention thus also concerns the use of a vector comprising a nucleic acid molecule as defined above. Preferably the vector is a replicative vector comprising on origin of replication (or autonomously replication sequence) that ensures multiplication of the vector in a suitable host for the vector. Alternatively a vector is capable of integrating into a host cell's genome, e.g. through homologous recombination or otherwise. A particularly preferred vector is an expression vector wherein a nucleotide molecule encoding a polypeptide as defined above, is operably linked to a promoter capable of directing expression of the coding sequence in a host cell for the vector.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues, such as preferably a monocyte or a macrophage cell or tissue derived there from.

Expression vectors allow a polypeptide of the invention as defined above to be prepared using recombinant techniques in which a nucleotide molecule encoding said polypeptide of interest is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra*); both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et aL (1985) Gene 34: 315 (describing cassette mutagenesis).

Typically, a nucleotide molecule encoding a desired polypeptide is used in an expression vector. The phrase "expression vector" generally refers to a nucleotide molecule represented by a nucleotide sequence that is capable of effecting expression of a gene in hosts compatible with such sequences. These expression vectors typically include at least suitable promoter sequences and optionally, transcription termination signals. Additional factors necessary or helpful in effecting expression can also be used as described herein. A nucleic acid or DNA encoding a polypeptide is incorporated into a DNA construct capable of introduction into and expression in an *in vitro* cell culture. Specifically, DNA constructs are suitable for replication in a prokaryotic host, such as bacteria, *e.g., E. coli,* or can be introduced into a cultured mammalian, plant, insect, e.g., Sf9, yeast, fungi or other eukaryotic cell lines.

DNA constructs prepared for introduction into a particular host typically include a replication system recognized by the host, the intended DNA segment encoding the desired polypeptide, and transcriptional and translational initiation and termination regulatory sequences operably linked to the polypeptide-encoding segment. A DNA segment is "operably linked" when it is placed into a functional relationship with another DNA segment. For example, a promoter or enhancer is operably linked to a coding sequence if it stimulates the transcription of the sequence. DNA for a signal sequence is operably linked to DNA encoding a polypeptide if it is expressed as a pre-protein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

The selection of an appropriate promoter sequence generally depends upon the host cell selected for the expression of the DNA segment. Examples of suitable promoter sequences include prokaryotic, and eukaryotic promoters well known in the art (see, e.g. Sambrook and Russell, 2001, *supra*). The transcriptional regulatory sequences typically include a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, *supra*). Expression vectors include the replication system and transcriptional and translational regulatory sequences together with the insertion site for the polypeptide encoding segment can be employed. Examples of workable combinations of cell lines and expression vectors are described in Sambrook and Russell (2001, *supra*) and in Metzger et aL (1988) Nature 334: 31-36. For example, suitable expression vectors can be expressed in, yeast, *e.g. S. cerevisiae, e.g.,* insect cells, *e.g.,* Sf9 cells, mammalian cells, *e.g.,* CHO cells and bacterial cells, *e.g., E. coli.* The host cells may thus be prokaryotic or eukarotic host cells. A host cell may be a host cell that is suitable for culture in liquid or on solid media. A host cell is preferably used in a method for producing a polypeptide of the invention as defined above or in a method for identification of a substance as defined herein. Said method comprises the step of culturing a host cell under conditions conducive to the expression of a polypeptide. Optionally the method may comprise recovery of a polypeptide. A polypeptide may e.g. be recovered from the culture medium by standard protein purification techniques, including a variety of chromatography methods known in the art per se.

Alternatively, a host cell is a cell that is part of a multi-cellular organism such as a transgenic plant or animal, preferably a non-human animal. A transgenic plant comprises in at least a part of its cells a vector as defined above. Methods for generating transgenic plants are e.g. described in U.S. 6,359,196 and in the references cited therein. Such transgenic plant or animal may be used in a method for producing a polypeptide of the invention as defined above and/or in a method for identification of a substance both as defined herein. For transgenic plant, the method comprises the step of recovering a part of a transgenic plant comprising in its cells the vector or a part of a descendant of such transgenic plant, whereby the plant part contains said polypeptide, and, optionally recovery of said polypeptide from the plant part. Such methods are also described in U.S. 6,359,196 and in the references cited therein. Similarly, the transgenic animal comprises in its somatic and germ cells a vector as defined above. The transgenic animal preferably is a non-human animal. More preferably, a non-human animal is a mouse. Methods for generating transgenic animals are e.g. described in WO 01/57079 and in the references cited therein. Such transgenic animals may be used in a method for producing a polypeptide as defined herein, said method comprising the step of recovering a body fluid from a transgenic animal comprising the vector or a female descendant thereof, wherein the body fluid contains said polypeptide, and, optionally recovery of said polypeptide from the body fluid. Such methods are also described in WO 01/57079 and in the references cited therein. The body fluid containing said polypeptide preferably is blood or more preferably milk.

Another method for preparing a polypeptide is to employ an *in vitro* transcription/translation system. DNA encoding a polypeptide is cloned into an expression vector as described *supra.* The expression vector is then transcribed and translated *in vitro.* The translation product can be used directly or first purified. A polypeptide resulting from *in vitro* translation typically does not contain the post-translation modifications present on polypeptides synthesised *in vivo,* although due to the inherent presence of microsomes some post-translational modification may occur. Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987.

In this document and in its claims, the verb "to comprise" and its conjugations is used in its non-limiting sense to mean that items following the word are included, but items not specifically mentioned are not excluded. In addition the verb "to consist" may be replaced by "to consist essentially of meaning that a polypeptide or a nucleic acid construct or an antibody as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one". Each embodiment as identified herein may be combined together unless otherwise indicated. All patent and literature references cited in the present specification are hereby incorporated by reference in their entirety.

The invention is further illustrated by the following examples which should not be construed for limiting the scope of the present invention.

### Description of the figures

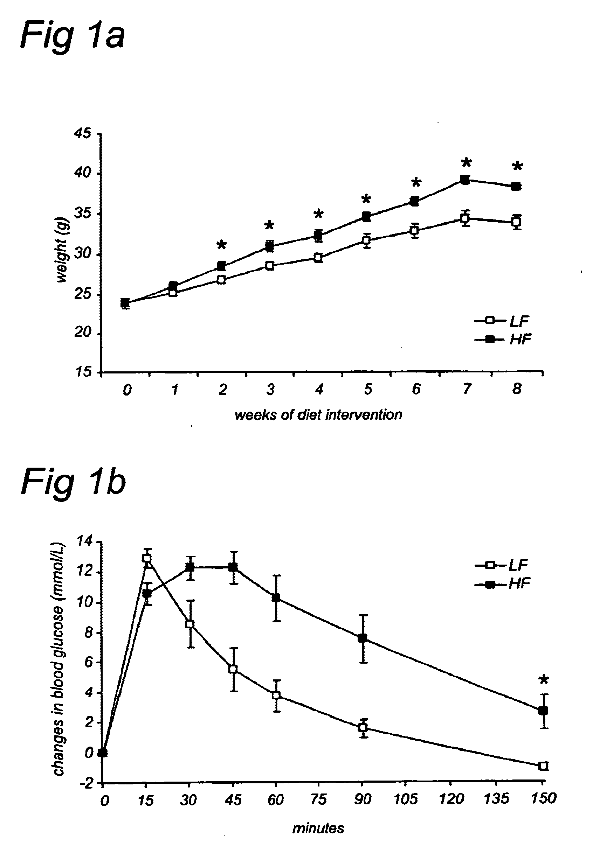

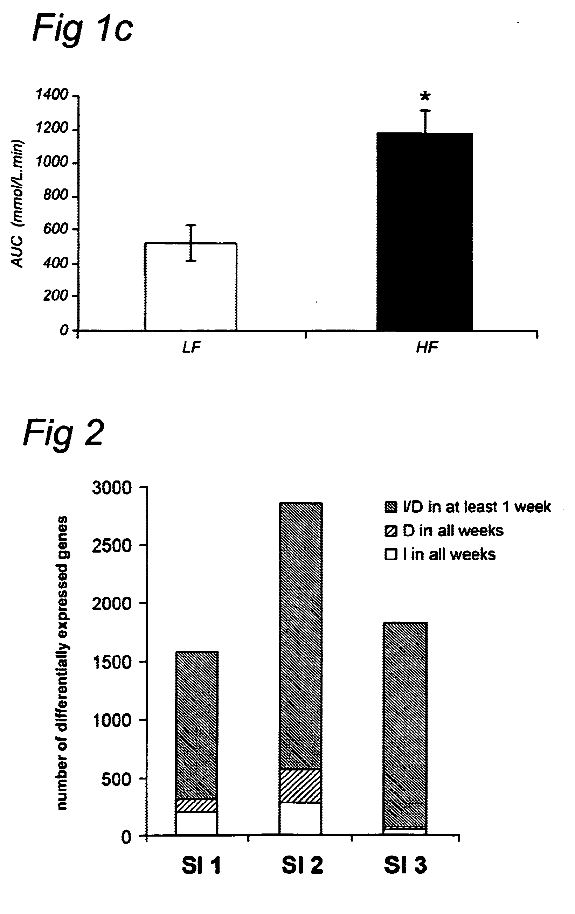

**Figure 1****. Body weight and oral glucose tolerance test**. (A) Body weight gain of C57BL/6J mice during a low-fat or high-fat diet intervention of 8 weeks. (B) and (C) An oral glucose tolerance test was performed after 7 weeks of diet intervention. After an oral gavage of 100 mg glucose, blood glucose levels were monitored for 150 minutes. The changes in blood glucose levels (B) and the area under he curve were calculated (C). In (A) and (B), data are means ± SEM. * p<0.05. LF = low-fat diet, HF = high-fat diet.
**Figure 2****. Dietary fat-induced differential gene expression along the longitudinal axis of the small intestine**. For the proximal (SI 1), middle (SI 2) and distal part of the small intestine (SI 3), the numbers of genes that are differentially expressed in at least one week of diet intervention are plotted (grey bars). Among those genes are genes that are consistently up- (I) or down-regulated (D) on a high-fat diet (white and black bars, respectively).

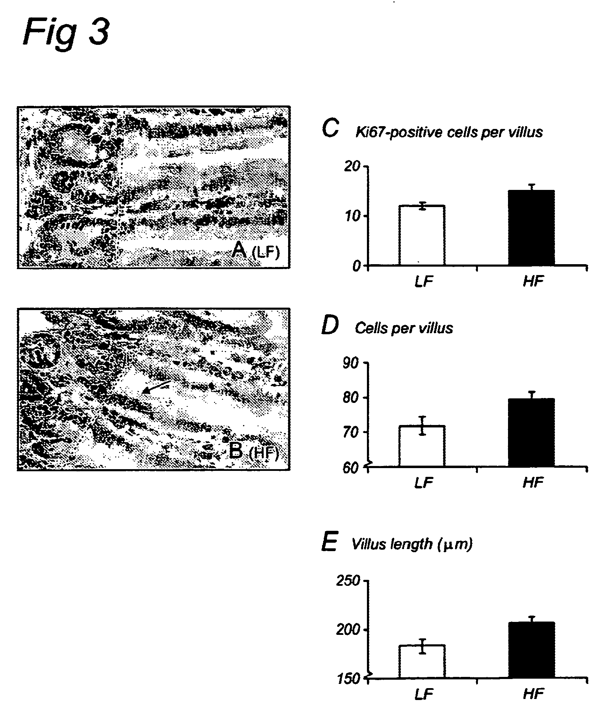

**Figure 3****. Immunohistochemical analysis of dietary fat-induced cell proliferation in the small intestine of C57BL/6J mice.** Immunohistochemistry was performed on distal small intestinal sections of C57BL/6J mice fed a low-fat (A) or high-fat diet (B) using Ki67-specific antibodies. The villus is defined from dotted line to top of the villus and the arrow indicates Ki67-specific staining (brown) at the bottom of the villus. Next to the number ofKi67-positive cells per villus (C), also the total number of villus cells (D) and villus length (E) were determined. Therefore, per mouse 15 villi were observed and the mean values were calculated. A-specific staining was detectable in the lamina propria due to cross-reactivity of the goat-anti-rat antibody (also seen in negative control without Ki67 antibodies, data not shown). * p<0.05. LF = low-fat diet, HF = high-fat diet.

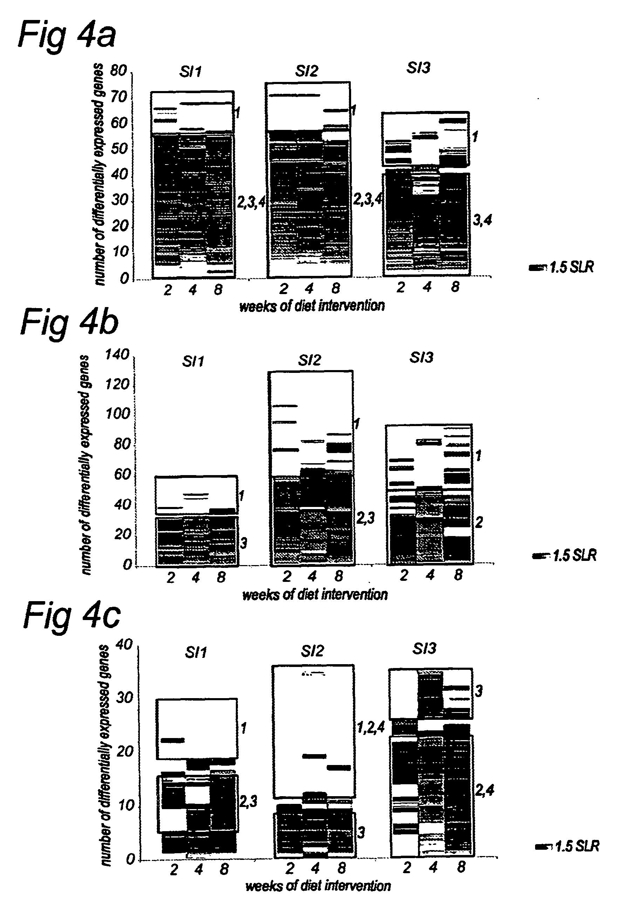

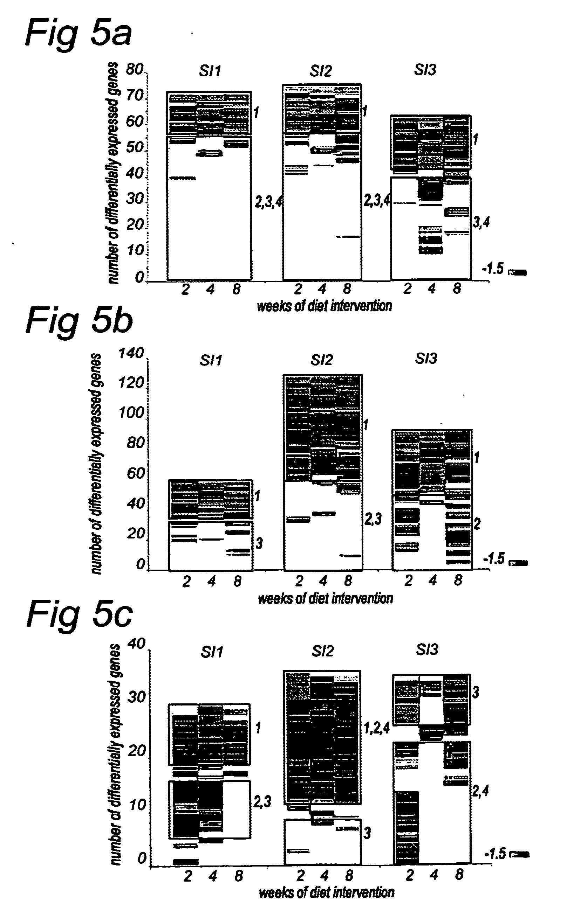

**Figures 4** **and** **5****. Heat map diagrams of differentially expressed genes on a high-fat diet.** SLR of differentially expressed genes related to lipid metabolism (A), cell cycle (B) and inflammation/immune response (C) are clustered in a heat map diagram for the proximal (SI 1), middle (SI 2) and distal part of the small intestine (SI 3). Figure 4 relates to the down-regulation of gene expression, whereas figure 5 relates to the up-regulation. Amongst other genes that display similar expression patterns on a high-fat diet, the boxes include differentially expressed genes that share association with particular biological processes (numbered). Differentially expressed genes with a -0.3 > SLR > 0.3 in at least one week of diet intervention are included and the color scheme ranges from SLR -1.5 to 1.5. Next to SLR, also the numbers of differentially expressed genes are visualized.

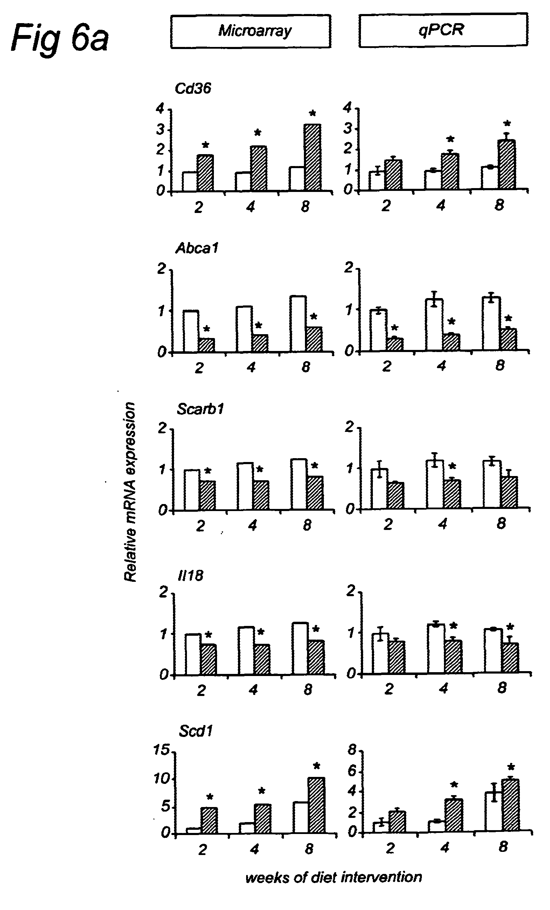

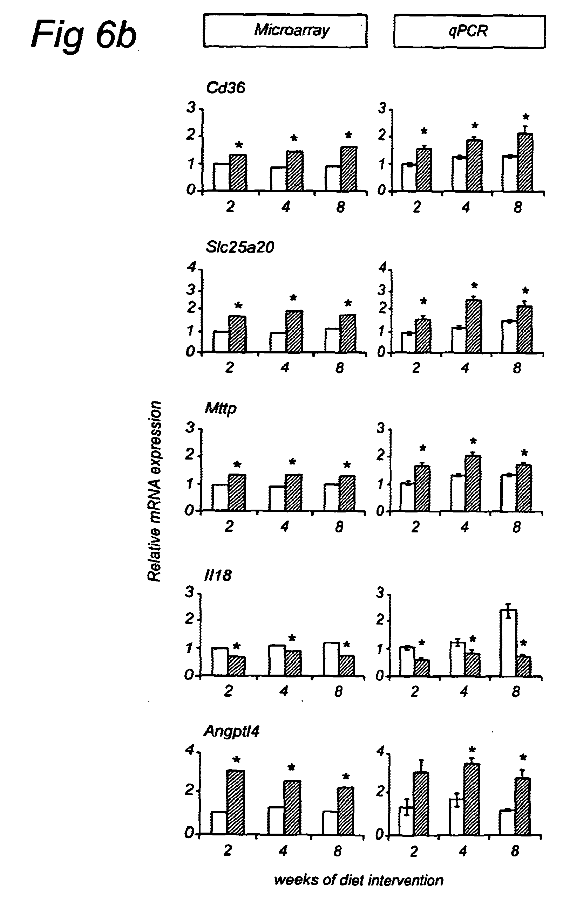

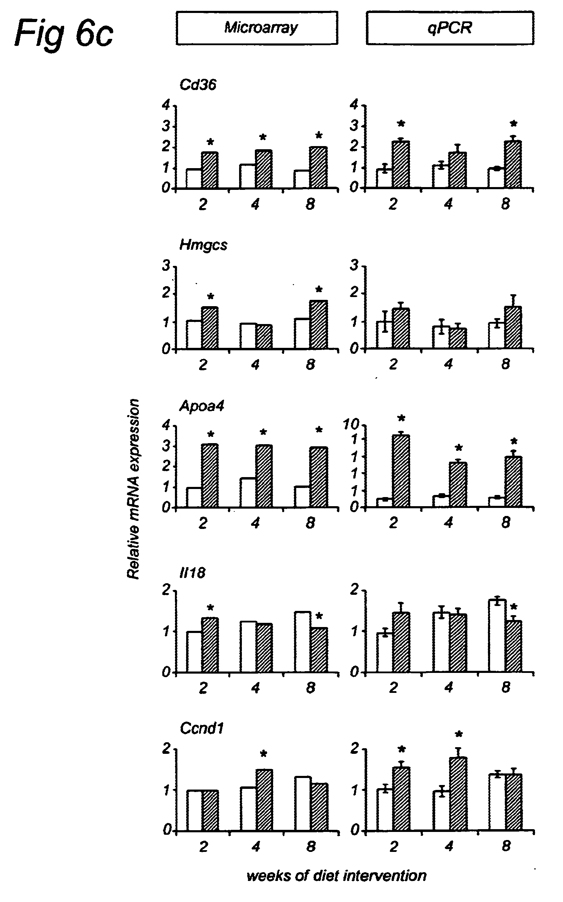

**Figure 6****. Verification of microarray results in individual mice by qPCR analysis.** For the proximal (A), middle (B) and distal part of the small intestine (C), five genes that were found to be differentially expressed by microarray analysis were randomly selected and their expression was validated in individual mouse samples by qPCR. Only the results of the 18S normalization are shown as they are representative for the results of the cyclophilin A normalization. The qPCR data are visualized as the mean expression of all individual mice per diet group per time point ± SEM, relative to the expression on the LF diet at week 2 which was set to 1. * p<0.05. LF = low-fat diet, HF = high-fat diet.

### Examples

### Materials & Methods

### Animals and diets

Male C57BL/6J mice were purchased from Harlan (Horst, The Netherlands) and were housed in the light- and temperature-controlled animal facility of Wageningen University. They had free access to water and prior to the diet intervention they received standard laboratory chow (RMH-B, Arie Blok BV, Woerden, The Netherlands). All experiments were approved by the Ethical Committee on animal testing of Wageningen University.

In this study we investigated the effect of dietary fat on development of obesity and insulin resistance and on small intestinal gene expression in C57BL/6J mice. After a run-in period of 3 weeks on the low-fat diet, 9 week old mice were fed a powder high- or a low-fat purified diet for 2, 4, and 8 weeks (n=6 per diet, per time point). Low-fat and high-fat diets are based on 'Research Diets' formulas D12450B / D12451, with adaptations regarding type of fat (palm oil in stead of lard) and carbohydrates to mimic the fatty acid and carbohydrate composition of the average human diet in Western societies (Research diet services, Wijk bij Duurstede, The Netherlands). The complete compositions of the diets are given in supplementary table S1. It should be noted that in these diets the energy density of all nutrients, except fat and starch, are equal. Body weight was recorded weekly and after 7 weeks of diet intervention an oral glucose tolerance test was performed. Therefore, after 6-hours fasting, mice received 0.5ml of a 20% glucose solution via an oral gavage and blood glucose was measured after 15, 30, 45, 60, 90 and 150 minutes using Accu-Chek blood glucose meters (Roche Diagnostics, Almere, The Netherlands). At the end of the experiment, mice were anaesthetized with a mixture of isofluorane (1.5%), nitrous oxide (70%) and oxygen (30%). The small intestines were excised and the adhering fat and pancreatic tissue were carefully removed. The small intestines were divided in three equal parts along the proximal to distal axis (SI 1 = proximal part; duodenum, SI 2 = middle part; jejunum and SI 3 = distal part; ileum). Small intestinal epithelial cells were scraped, snap-frozen in liquid nitrogen, and stored at -80 °C until RNA isolation. For immunohistochemical analysis, a similar low-fat and high-fat diet intervention study was performed for 2 weeks (n=12 per diet). Small intestines were again excised, divided in three equal parts, cut open longitudinally, and washed with PBS. Thereafter, the small intestinal parts were fixed in 10% buffered formalin and imbedded in paraffin as 'Swiss rolls'.

### RNA isolation

Total RNA was isolated using TRIzol reagent (Invitrogen, Breda, The Netherlands) according to the manufacturer's instructions. The isolated RNA was further column-purified using the SV total RNA isolation system (Promega, Leiden, The Netherlands). RNA concentration was measured on a NanoDrop ND-1000 UV-Vis spectrophotometer (Isogen, Maarssen, The Netherlands) and analyzed on a bioanalyzer (Agilent Technologies, Amsterdam, the Netherlands) with 6000 Nano Chips according to the manufacturer's instructions.

### Microarray hybridization and analysis

For each part of the small intestine, total RNA was pooled per diet group and per time point (n=6). RNA was hybridized to Mouse genome 430 2.0 arrays (Affymetrix, Santa Clara, CA, USA). Detailed methods for the labelling and subsequent hybridizations to the arrays are described in the eukaryotic section in the GeneChip Expression Analysis Technical Manual Rev. 3 from Affymetrix. Arrays were scanned on an Affymetrix GeneChip Scanner 3000. Data analysis was performed using Microarray Analysis Suite 5.0 (MAS 5.0). To estimate the magnitude and direction of differential gene expression for the high-fat versus low-fat treatments, MAS 5.0 software provides signal log ratio's (SLR). If the SLR is equal to or greater than 0, fold change is obtained with +2^{SLR} otherwise with -2^{-SLR}. Array data have been submitted to the Gene Expression Omnibus, accession number GSE8582.

To determine overrepresentation of Gene Ontology (GO) Biological Process subsets upon high-fat diet intervention, an ErmineJ overrepresentation analysis (ORA) was performed (33). Gene score files that were used as input contained the 'change p-values' of all probes sets provided by MAS 5.0 comparison analysis. By setting the Gene score threshold to 0.0025, only significantly differentially expressed genes were included in the ORA analyses. Moreover, only GO subsets that contained between 2 and 125 genes were taking into account. The ErmineJ software generally uses Benjamini-Hochberg correction of p-values to determine which gene sets are selected with a particular false discovery rate (FDR). The FDR is considered a rapid and reasonable guide to which gene sets are likely to be of highest interest.

Heat map diagrams visualizing SLR of differentially expressed genes are made using Spotfire DecisionSite® software by applying hierarchical clustering.

### cDNA synthesis and real-time quantitative PCR

Single-stranded complementary DNA (cDNA) was synthesized from 1 µg of total RNA using the Reverse transcription system (Promega, Leiden, The Netherlands) following the supplier's protocol. cDNA was PCR amplified with Platinum Taq DNA polymerase (all reagents were from Invitrogen). Primer sequences that we used for real-time quantitative PCR reaction (qPCR) were chosen based on the sequences available in the GenBank database (www.ncbi.nlm.nih.gov) and these are listed in supplementary table S2. qPCRs was performed using SYBR green and a MyIQ thermal cycler (Bio-Rad laboratories BV, Veenendaal, The Netherlands). The following thermal cycling conditions were used: 2 min at 94°C, followed by 40 cycles of 94°C for 15 s and 60°C for 45 s. PCR reactions were performed in duplicate and all samples were normalized to 18S and cyclophilin A expression.

### Immunohistochemistry

Four-micrometer sections of paraffin-embedded distal part of the small intestine were mounted on Superfrost microscope slides. These sections were dewaxed in xylene and rehydrated in a series of graded alcohols. To block endogenous peroxidase activity, slides were incubated with 3% H₂O₂ for 20 minutes. Antigen retrieval is performed by placing the slides in citrate buffer (pH 6.0) and heat them in a microwave oven 5 min 700 W (without lid) and 4 times 5 min 500 W (with lid). After cooling down to room temperature, the sections were briefly washed with PBS. Prior to staining, a 20 minutes preincubation was performed using 20% normal goat serum (Vector Laboratories, Burlingame, CA, USA). The sections were stained in a three-step procedure utilizing the following incubations: overnight incubation at 4°C with rat monoclonal antibodies against Ki67 (Clone TEC-3) (DakoCytomation B.V., Heverlee, Belgium), diluted 1:200 in PBS. Thereafter, the sections were incubated with a biotinylated goat-anti-rat for 30 minutes, followed by 45 minutes incubation with peroxidase-labelled avidin-biotin complex (Vector Laboratories). Between all incubations, sections were washed three times in PBS. Diaminobenzidine tetrahydrochloride (DAB, Vector Laboratories) was used as substrate to visualize the bound antibodies. After counterstaining with Meyer's hematoxylin, sections were mounted with DePex mounting medium (Gurr, BDH, Poole, Dorset, UK).

### Statistical analysis

All data are reported as the mean ± SEM. The differences between the mean values were tested for statistical significance by the two-tailed Student's t test.

### Results

### Dietary fat-induced obesity and insulin resistance in C57BL/6J mice

To evaluate the effect of a high-fat diet on the development of obesity and insulin resistance, C57BL/6J mice were fed a high-fat versus low-fat diet for eight weeks. Figure 1A shows that already after two weeks on the high-fat diet, C57BL/6J mice demonstrate a significantly higher weight gain than mice on the low-fat diet. Moreover, an oral glucose tolerance test performed after seven weeks of diet intervention showed that the high-fat diet induced a significantly higher glucose intolerance (Figure 1B), indicating development of insulin resistance. As the intake of the high-fat and low-fat diets was isoenergetic, these data indicate that dietary fat (palm oil) induces obesity and insulin resistance in C57BL/6J mice.

### Dietary fat-induced changes in small intestinal gene expression

After 2, 4 and 8 weeks of diet intervention, C57BL/6J mice were sacrificed (n=6 per diet, per time point), small intestines were isolated and divided into three equal parts along the proximal to distal axis. For each part of the small intestine, dietary fat-induced differential gene expression was analyzed and the numbers of genes that are differentially expressed in at least one week of diet intervention are visualized in figure 2. The consistently differentially expressed genes with a fold change lower than -3 and higher than +3 are listed in supplementary table S3. The highest numbers of genes, transiently as well as sustained differentially expressed during diet intervention, are found in the middle part of the small intestine, indicating that the effect of dietary fat on gene expression is most pronounced in the jejunum.

As microarray analyses in this study were performed on mouse samples that were pooled per diet group per time point, qPCR was used to verify differential gene expression of randomly selected genes in individual mouse samples. qPCR results were highly in accordance with the microarray data (supplementary figure S1), especially in the middle part of the small intestine. In the proximal and distal part of the small intestine, differential expression of some genes did not reach significance by qPCR, due to a higher variance among individual mouse samples.

### Biological processes influenced by dietary fat in the small intestine

To determine which biological processes in the small intestine are highly influenced by dietary fat, we performed an overrepresentation analysis (ORA) for each part of the small intestine, including all genes showing differential expression in at least one week of diet intervention. The false discovery rate (FDR) that is calculated in the ORA analysis is considered a rapid and reasonable guide to which gene sets are likely to be of highest interest (33). Table 1 displays the GO Biological Process subsets that are overrepresented in the different parts of the small intestine (FDR < 0.01) in mice fed a high-fat diet. Additionally, in figure 3, heat map diagrams illustrate the numbers of genes that are annotated to the GO terms listed in table 1 and the magnitude and direction of their differential gene expression indicated by the SLR.

ORA analysis reveals that biological processes related to lipid metabolism are highly regulated by dietary fat in all parts of the small intestine. Additionally, the heat map diagrams in figure 3A show that the up- or down-regulation of many lipid metabolism related genes is very consistent in time and that the strongest dietary fat effects can be observed in the proximal and middle part of the small intestine. In these parts, genes involved in fatty acid transport, chylomicron synthesis and especially fatty acid oxidation are highly up-regulated, whereas genes involved in cholesterol transport are down-regulated by dietary fat. In the distal part of the small intestine, similar regulation of fatty acid oxidation and cholesterol transport is seen, but less prominent than in the more proximal parts of the small intestine. These data indicate that dietary fat processing/handling is mainly accomplished in the duodenum and jejunum. However, the ileum is still capable of handling the overflow of fat.

Additionally, ORA analysis and heat map diagrams (figure 3B) show the effect of dietary fat on regulation of cell cycle related processes, which is most pronounced in the middle and distal parts of the small intestine. Cell proliferation seems to be enhanced by dietary fat early in diet intervention, as genes that are essential for progression through cell cycle are up-regulated in the first weeks and genes involved in apoptosis are down-regulated. Remarkably, after 8 weeks of diet intervention, hardly any differential gene expression related to cell cycle can be detected. To ensure that the dietary fat-induced modulations in cell cycle related processes reflect proliferation of enterocytes and not immune cells, we performed immunohistochemistry on the distal part of the small intestine using Ki67-specific antibodies (figure 4). Differences in Ki67-staining of the small intestines exposed to the low-fat or high-fat diet were most pronounced at the bottom of the villi (Figure 4A and 4B). Although this increase in Ki67-positive cells per villus did not reach significance (p=0.07), villus length and the total number of cells per villus were significantly higher in mice fed the high-fat diet. These data indicate that cell proliferation induced by dietary fat results in enlargement of small intestinal villi.

Biological Process subsets related to inflammation/immune response are also overrepresented in small intestine after feeding a high-fat diet. In the proximal and distal small intestine, the differential expression of genes related to these processes is not very consistent in time. However, in the middle part of the small intestine, a substantial number of genes show a sustained down-regulation throughout high-fat diet intervention. Remarkably, many of these down-regulated genes are known to be interferon gamma (IFNγ)-inducible genes (28, 42). Despite this consistent down-regulation of genes in the jejunum, which suggests a diminished inflammatory disposition after exposure to elevated levels of dietary fat, it remains difficult to draw a definitive conclusion on inflammatory status of the overall small intestine, as hardly any gene shows a consistent up- or down-regulation in all parts of the small intestine. Taken together, ORA analysis showed that dietary fat highly influences processes related to lipid metabolism, proliferation and inflammation and/or immune response in the small intestine of C57B1/6J mice.

### Dietary fat-induced gene expression changes of small intestinal secreted proteins

In response to dietary components, the small intestinal mucosa can be triggered to secrete signaling proteins that are able to induce systemic effects, such as modulation of metabolism in peripheral organs. To identify secreted proteins that are differentially expressed during high-fat diet intervention, we performed a secretome analysis. For this analysis, genes that were differentially expressed in at least one week of diet intervention (fold change >1.5) were additionally selected for their corresponding GO Cellular Component term 'extracellular region/space' (GO:0005576/GO:0005615) (Table 2). Some of these selected genes showed a consistent differential gene expression throughout the small intestine (e.g. Angpt14, ApoC2, Dnase1, Cgref1, Gas6, H2-Q 10), whereas for other genes the changes were more restricted to a particular part of the gut (e.g. Cck, Igfbp3, Regl, Fgf15, Cc128, Cc15, Pyy). For several genes, also a time effect could be detected, as they were showing early (e.g. Igfbp4, Ttr) or late phase (e.g. Cc15, Cc128, Igj, Fgf15) responses. Consistent with the ORA analysis data described above, many of the secreted proteins are related to lipid metabolism, especially chylomicron synthesis (e.g. ApoA4, ApoC2, ApoC3), and inflammation/immune response (e.g. several chemokines, H2-Q10,Il18, Mif, Rsad2, Saa1/2). Although we do not propose that all of these secreted proteins act as signaling molecules that provoke a systemic effect on peripheral organs, we consider genes related to inflammation/immune response and the gut hormones (e.g. Cck, Pyy) as promising candidates. Also genes with a pronounced differential gene expression, of which function is not completely elucidated yet (e.g. Angpt14, Oit1, Smpd13a/b) are potential interesting signaling molecules that might contribute to development of obesity and/or insulin resistance. Table 4 identifies the same genes as table 2, gives their tissue distribution and their level of expression. In grey in table 4, 15 genes are selected for their high level of expression in the animal model and their restricted tissue distribution (restricted till specific gastro-intestinal tract). Each of these genes alone or in combination are attractive to be used as markers in the present invention since their gene product is secreted into the serum, their expression level is up-regulated in the studied animal model and they are expressed in a limited number of tissues. The 15 genes of Table 4 are further identified in Table 5. As Fam3D (Oit1) and ApoA4 are gut-specific markers (small intestine), their differences in gene expression as measured in serum may easily be extrapolated to differences in gene expression in the small intestine.

### Discussion

In this study, we demonstrated that C57BL/6J mice develop obesity and glucose intolerance on a high-fat diet that mimics the fatty acid composition of a Western-style human diet. Microarray analysis showed that dietary fat induces a substantial number of changes in gene expression throughout the small intestine. However, the most pronounced effects were detectable in the middle part of the small intestine. Biological processes that we found to be highly influenced in the small intestine by feeding a high-fat diet are predominantly associated with lipid metabolism, inflammation/immune response and cell cycle.

Lipid metabolism related genes, especially Ppara target genes involved in fatty acid transport and fatty acid oxidation, were highly and consistently regulated by dietary fat. This indicates that lipid metabolism related processes are presumably very important for efficient dietary fat handling in the small intestine. Kondo et al. recently compared the gene expression levels of several Pparα target genes involved in fatty acid catabolism between obesity-resistant A/J versus obesity-prone C57B1/6J mice after feeding a high-fat diet (27). In their study, the basal as well as the dietary fat-induced up-regulated expression of the genes were higher in the A/J mice compared to the C57B1/6J mice. They suggested that in C57B1/6J mice fatty acid catabolism in the small intestine proceeds less efficient than in A/J mice and that an impaired activation of Ppara might play an important role in this process. Moreover, the highly reduced expression level of Cyp4a10 in C57B1/6J mice suggested that ω-oxidation plays an essential role in the diminished efficacy of small intestinal fatty acid handling. The ω-oxidation is known to be a compensatory mechanism when β-oxidation is not sufficient, which seems to be the case on a high-fat diet intervention. Interestingly, it was previously shown that also Ppargc1a is involved in fatty acid oxidation, as together with Ppara it can cooperatively induce the expression of Ppara target genes and increase cellular fatty acid oxidation rates (49). Moreover, a decreased expression of Ppargc1a was linked to an inefficient fatty acid oxidation and associated with an impaired glucose tolerance in mice fed a high-fat diet (29). Based on these studies, we speculate that in the C57B1/6J mice the dietary fat-induced down-regulation of Ppargc1a is related to a suboptimal activation of Ppara. This results in an inefficient fatty acid oxidation in the small intestine, in which we believe ω-oxidation has a pronounced role. How this impaired fatty acid handling in the small intestine might contribute to development of dietary fat-induced obesity and/or insulin resistance is not yet known and has to be investigated in future studies.

Next to a role in lipid handling, Ppars are also known to be related to inflammation and immune response. Activated Ppars can suppress production of pro-inflammatory cytokines or related mediators, such as tumor necrosis factor α (Tnfα) (20), IFNγ (18) and nuclear factor kappa B (Nfkb) (10). So, lipids can regulate inflammatory and immune processes via Ppars and this might explain the down-regulation of IFNγ-inducible genes in the middle part of the small intestine, which was found to be most susceptible to dietary fat-induced gene expression changes. On the other hand, the down-regulation of IFNγ-inducible genes can be the result of the decreased expression of pro-inflammatory cytokine Il18, which actions are mediated by IFNγ. Interestingly, a recent study showed that Il18 null mice have markedly increased body weight and are insulin resistant (39). It is even suggested that I118 possesses a glucose-lowering potential. Based on our data, we hypothesize that this recently proposed role of Il18 in obesity and insulin resistance is mediated via the IFNγ signaling pathway.

There is growing evidence that chronic inflammation contributes to development of obesity and insulin resistance (21). Some of these inflammatory pathways, which are most extensively studied in liver, adipose tissue and muscle, involve toll-like receptor 4 (Tlr4) (44), tumor necrosis factor α (Tnfα) (22), nuclear factor kappa B (Nfkb) (5), Jun kinases (Jnk) and insulin receptor substrates (Irs) (22, 47). Disturbances in these pathways can lead to disruption of insulin action/signaling and thereby affecting insulin sensitivity. However, as insulin signaling is not very likely in the small intestine, due to lack of insulin receptors, these inflammation pathways are not expected to contribute to the role of the small intestine in development of obesity and insulin resistance. In our microarray data, we could indeed not detect any expression of Tnfα, Tlr4 and Irs and no differential gene expression of Nfkβ and Jun kinases.

Furthermore, our data showed that in the first weeks of high-fat diet intervention, cell proliferation is enhanced in the middle and distal part of the small intestine, leading to an increase in villus cell number and villus length. Petit et al, recently also reported enhanced proliferation in jejunum after feeding mice a high-fat diet, even though their diet had a somehow different fatty acid composition than was used in our study (40). The dietary fat-induced enlargement of the villi might be functional to extent the capacity of fat absorption. Remarkably, we found that the dietary fat-induced cell proliferation was attenuated after a longer period of diet intervention. This suggests that the increased uptake capacity reaches a certain maximum between 4 and 8 weeks on a high-fat diet, which might finally result in an inefficient absorption and processing of dietary fat. Although hardly any gene related to cell cycle in the small intestine was previously described to be associated with obesity and/or insulin resistance, our data indicate that small intestinal cell proliferation is important for an optimal functioning of the small intestine when exposed to a high-fat diet.

As signaling molecules secreted by the small intestine are able to induce systemic effects by influencing metabolic homeostasis in peripheral organs, inefficient or altered regulation of these molecules might be related to the etiology of obesity and/or insulin resistance (9, 12). Various studies describe the potential role of gut hormones in metabolic syndrome related disease states. For the incretin hormones Gip and Glp1, which can induce a systemic effect on glucose homeostasis, our data imply that elevated levels of the bioactive compounds are available on a high-fat diet. As previous studies showed that increased plasma levels of Gip and Glp1 even lead to an improved insulin sensitivity (36, 37), it is not very likely that the incretins contribute to development of obesity and glucose intolerance in our C57BL/6J mouse model. Other secreted proteins that are more likely to provide a substantial contribution to small intestinal involvement are Il18, Ffgf15, Mif and Igfbp3. Their differential expression in the small intestine induced by dietary fat corroborates results of previous knock-out and over-expression studies showing association with obesity and/or insulin resistance (7, 14, 39, 45). Contradictory to previous studies showing that suppression of Angptl4 mediated by gut-microbiota was related to dietary fat-induced obesity (2), we found a sustained up-regulation of this gene in all parts of the small intestine. This implies that normal suppression of Angptl4 by gut microbiota (1) is consistently counteracted by dietary fat. As despite this persistent up-regulation of Angptl4, C57B1/6J mice still became obese on a high fat diet, we conclude that small intestinal Angptl4 is probably not a main contributor to development of obesity. Interestingly, however, studies of Mandard et al. showing that elevated levels of Angptl4 are related to glucose intolerance might indicate that the dietary-fat induced up-regulation of Angptl4 in the small intestine can provoke a systemic effect on development of insulin resistance (35). Further research will be required to more accurately elucidate the function of Angptl4 in the small intestine and its potential involvement in metabolic syndrome.

In summary, we found that a high-fat diet that mimics the fatty acid composition of a Western-style human diet induces obesity and insulin resistance in C57BL/6J mice. The biological processes that are most apparently modulated in the small intestine by this dietary fat are related to lipid metabolism, cell cycle and inflammation/immune response. Additionally, secretome analysis revealed several secreted proteins with a modulated expression on a high fat diet that might provoke metabolic effects in liver, muscle and adipose tissue. As many of the genes, showing dietary fat-induced changes, were previously already linked to obesity and/or insulin resistance, this exploratory study provides several leads for an essential role of the small intestine in the etiology of these disease states. To narrow down the small intestinal contribution to development of metabolic syndrome, future research with a special focus on efficacy of fatty acid catabolism and function of small intestinal secreted proteins such as Il18, Fgf15, Mif, Igfbp3 and Angptl4 will be done.

### References

**1.** Backhed F, Ding H, Wang T, Hooper LV, Koh GY, Nagy A, Semenkovich CF, and Gordon JI. The gut microbiota as an environmental factor that regulates fat storage. PNAS 101: 15718-15723, 2004.
2. Backhed F, Manchester JK, Semenkovich CF, and Gordon JI. From the Cover: Mechanisms underlying the resistance to diet-induced obesity in germ-free mice. PNAS 104: 979-984, 2007.
3. Bonen A, Tandon NN, Glatz JFC, Luiken JJFP, and Heigenhauser GJF. The fatty acid transporter FAT//CD36 is upregulated in subcutaneous and visceral adipose tissues in human obesity and type 2 diabetes. Int J Obes 30: 877, 2006.
4. Bunger M, van den Bosch HM, van der Meijde J, Kersten S, Hooiveld GJ, and Muller M. Genome-wide analysis of PPAR{alpha} activation in murine small intestine. Physiol Genomics 2007.
5. Cai D, Yuan M, Frantz DF, Melendez PA, Hansen L, Lee J, and Shoelson SE. Local and systemic insulin resistance resulting from hepatic activation of IKK-[beta] and NF-[kappa]B. Nat Med 11: 183, 2005.
6. Campion J, Milagro FI, Fernandez D, and Martinez JA. Diferential gene expression and adiposity reduction induced by ascorbic acid supplementation in a cafeteria model of obesity. J Physiol Biochem 62: 71-80, 2006.
7. Church TS, Willis MS, Priest EL, Lamonte MJ, Earnest CP, Wilkinson WJ, Wilson DA, and Giroir BP. Obesity, macrophage migration inhibitory factor, and weight loss. Int J Obes (Lond) 29: 675-681, 2005.
8. Conarello SL, Li Z, Ronan J, Roy RS, Zhu L, Jiang G, Liu F, Woods J, Zycband E, Moller DE, Thornberry NA, and Zhang BB. Mice lacking dipeptidyl peptidase IV are protected against obesity and insulin resistance. PNAS 100: 6825-6830, 2003.
9. Cummings DE, and Overduin J. Gastrointestinal regulation of food intake. J Clin Invest 117: 13-23, 2007.
10. Delerive P, Fruchart JC, and Staels B. Peroxisome proliferator-activated receptors in inflammation control. J Endocrinol 169: 453-459, 2001.
11. Drucker DJ. Enhancing the Action of Incretin Hormones: A New Whey Forward? Endocrinology 147: 3171-3172, 2006.
12. Drucker DJ. The role of gut hormones in glucose homeostasis. J Clin Invest 117: 24-32, 2007.
13. Duran-Sandoval D, Cariou B, Fruchart J-C, and Staels B. Potential regulatory role of the farnesoid X receptor in the metabolic syndrome. Biochimie 87: 93, 2005.
14. Fu L, John LM, Adams SH, Yu XX, Tomlinson E, Renz M, Williams PM, Soriano R, Corpuz R, Moffat B, Vandlen R, Simmons L, Foster J, Stephan J-P, Tsai SP, and Stewart TA. Fibroblast Growth Factor 19 Increases Metabolic Rate and Reverses Dietary and Leptin-Deficient Diabetes. Endocrinology 145: 2594-2603, 2004.
15. Gertow K, Pietilainen KH, Yki-Jarvinen H, Kaprio J, Rissanen A, Eriksson P, Hamsten A, and Fisher RM. Expression of fatty-acid-handling proteins in human adipose tissue in relation to obesity and insulin resistance. Diabetologia 47: 1118-1125, 2004.
16. Guerre-Millo M, Gervois P, Raspe E, Madsen L, Poulain P, Derudas B, Herbert J-M, Winegar DA, Willson TM, Fruchart J-C, Berge RK, and Staels B. Peroxisome Proliferator-activated Receptor alpha Activators Improve Insulin Sensitivity and Reduce Adiposity. J Biol Chem 275: 16638-16642, 2000.
17. Gutierrez-Juarez R, Pocai A, Mulas C, Ono H, Bhanot S, Monia BP, and Rossetti L. Critical role of stearoyl-CoA desaturase-1 (SCD1) in the onset of diet-induced hepatic insulin resistance. J Clin Invest 116: 1686-1695, 2006.
18. Hara Y, Miura S, Komoto S, Inamura T, Koseki S, Watanabe C, Hokari R, Tsuzuki Y, Ogino T, Nagata H, Hachimura S, Kaminogawa S, and Ishii H. Exposure to fatty acids modulates interferon production by intraepithelial lymphocytes. Immunol Lett 86: 139-148, 2003.
19. Herder C, Kolb H, Koenig W, Haastert B, Muller-Scholze S, Rathmann W, Holle R, Thorand B, and Wichmann HE. Association of Systemic Concentrations of Macrophage Migration Inhibitory Factor With Impaired Glucose Tolerance and Type 2 Diabetes: Results from the Cooperative Health Research in the Region of Augsburg, Survey 4 (KORA S4). Diabetes Care 29: 368-371, 2006.
20. Hill MR, Clarke S, Rodgers K, Thornhill B, Peters JM, Gonzalez FJ, and Gimble JM. Effect of Peroxisome Proliferator-Activated Receptor Alpha Activators on Tumor Necrosis Factor Expression in Mice during Endotoxemia. Infect Immun 67: 3488-3493, 1999.
21. Hotamisligil GS. Inflammation and metabolic disorders. Nature 444: 860-867, 2006.
22. Hotamisligil GS, Peraldi P, Budavari A, Ellis R, White MF, and Spiegelman BM. IRS-1-Mediated Inhibition of Insulin Receptor Tyrosine Kinase Activity in TNF-alpha- and Obesity-Induced Insulin Resistance. Science 271: 665-670, 1996.
23. Jiang T, Wang Z, Proctor G, Moskowitz S, Liebman SE, Rogers T, Lucia MS, Li J, and Levi M. Diet-induced Obesity in C57BL/6J Mice Causes Increased Renal Lipid Accumulation and Glomerulosclerosis via a Sterol Regulatory Element-binding Protein-1c-dependent Pathway. J Biol Chem 280: 32317-32325, 2005.
24. Kadowaki T, Hara K, Yamauchi T, Terauchi Y, Tobe K, and Nagai R. Molecular Mechanism of Insulin Resistance and Obesity. Experimental Biology and Medicine 228: 1111-1117, 2003.
25. Kahn BB, and Flier JS. Obesity and insulin resistance. J Clin Invest 106: 473-481,2000.
26. Kalaany NY, Gauthier KC, Zavacki AM, Mammen PPA, Kitazume T, Peterson JA, Horton JD, Garry DJ, Bianco AC, and Mangelsdorf DJ. LXRs regulate the balance between fat storage and oxidation. Cell Metabolism 1: 231, 2005.
27. Kondo H, Minegishi Y, Komine Y, Mori T, Matsumoto I, Abe K, Tokimitsu I, Hase T, and Murase T. Differential regulation of intestinal lipid metabolism-related genes in obesity-resistant A/J vs. obesity-prone C57BL/6J mice. Am J Physiol Endocrinol Metab 291: E1092-1099, 2006.
28. Kota RS, Rutledge JC, Gohil K, Kumar A, Enelow RI, and Ramana CV. Regulation of gene expression in RAW 264.7 macrophage cell line by interferon-[gamma]. Biochemical and Biophysical Research Communications 342: 1137, 2006.
29. Koves TR, Li P, An J, Akimoto T, Slentz D, Ilkayeva O, Dohm GL, Yan Z, Newgard CB, and Muoio DM. Peroxisome Proliferator-activated Receptor-{gamma} Co-activator 1 {alpha}-mediated Metabolic Remodeling of Skeletal Myocytes Mimics Exercise Training and Reverses Lipid-induced Mitochondrial Inefficiency. J Biol Chem 280: 33588-33598, 2005.
30. Kreeft AJ, Moen CJA, Porter G, Kasanmoentalib S, Sverdlov R, van Gorp PJ, Havekes LM, Frants RR, and Hofker MH. Genomic analysis of the response of mouse models to high-fat feeding shows a major role of nuclear receptors in the simultaneous regulation of lipid and inflammatory genes. Atherosclerosis 182: 249, 2005.
31. Laaksonen DE, Niskanen L, Lakka HM, Lakka TA, and Uusitupa M. Epidemiology and treatment of the metabolic syndrome. Ann Med 36: 332-346, 2004.
32. Lee C-H, Olson P, Hevener A, Mehl I, Chong L-W, Olefsky JM, Gonzalez FJ, Ham J, Kang H, Peters JM, and Evans RM. PPAR{delta} regulates glucose metabolism and insulin sensitivity. PNAS 103: 3444-3449, 2006.
33. Lee H, Braynen W, Keshav K, and Pavlidis P. ErmineJ: Tool for functional analysis of gene expression data sets. BMC Bioinformatics 6: 269, 2005.
34. Lin J, Yang R, Tarr PT, Wu P-H, Handschin C, Li S, Yang W, Pei L, Uldry M, Tontonoz P, Newgard CB, and Spiegelman BM. Hyperlipidemic Effects of Dietary Saturated Fats Mediated through PGC-1[beta] Coactivation of SREBP. Cell 120:261,2005.
35. Mandard S, Zandbergen F, van Straten E, Wahli W, Kuipers F, Muller M, and Kersten S. The fasting-induced adipose factor/angiopoietin-like protein 4 is physically associated with lipoproteins and governs plasma lipid levels and adiposity. J Biol Chem 281: 934-944, 2006.
36. Marguet D, Baggio L, Kobayashi T, Bernard AM, Pierres M, Nielsen PF, Ribel U, Watanabe T, Drucker DJ, and Wagtmann N. Enhanced insulin secretion and improved glucose tolerance in mice lacking CD26. Proc Natl Acad Sci U S A 97: 6874-6879, 2000.
37. Mitani H, Takimoto M, Hughes TE, and Kimura M. Dipeptidyl peptidase IV inhibition improves impaired glucose tolerance in high-fat diet-fed rats: study using a Fischer 344 rat substrain deficient in its enzyme activity. Jpn J Pharmacol 88: 442-450, 2002.
38. Moller DE, and Kaufman KD. METABOLIC SYNDROME: A Clinical and Molecular Perspective. Annual Review of Medicine 56: 45-62, 2005.
39. Netea MG, Joosten LA, Lewis E, Jensen DR, Voshol PJ, Kullberg BJ, Tack CJ, van Krieken H, Kim SH, Stalenhoef AF, van de Loo FA, Verschueren I, Pulawa L, Akira S, Eckel RH, Dinarello CA, van den Berg W, and van der Meer JW. Deficiency of interleukin-18 in mice leads to hyperphagia, obesity and insulin resistance. Nat Med 12: 650-656, 2006.
40. Petit V, Arnould L, Martin P, Monnot MC, Pineau T, Besnard P, and Niot I. Chronic high-fat diet affects intestinal fat absorption and postprandial triglyceride levels in the mouse. J Lipid Res 48: 278-287, 2007.
41. Rahman SM, Dobrzyn A, Dobrzyn P, Lee S-H, Miyazaki M, and Ntambi JM. Stearoyl-CoA desaturase 1 deficiency elevates insulin-signaling components and down-regulates protein-tyrosine phosphatase 1B in muscle. PNAS 100: 11110-11115, 2003.
42. Rhee SJ, Walker WA, and Cherayil BJ. Developmentally Regulated Intestinal Expression of IFN-{gamma} and Its Target Genes and the Age-Specific Response to Enteric Salmonella Infection. J Immunol 175: 1127-1136, 2005.
43. Roche HM, Phillips C, and Gibney MJ. The metabolic syndrome: the crossroads of diet and genetics. Proc Nutr Soc 64: 371-377, 2005.
44. Shi H, Kokoeva MV, Inouye K, Tzameli I, Yin H, and Flier JS. TLR4 links innate immunity and fatty acid-induced insulin resistance. J Clin Invest 116: 3015-3025, 2006.
45. Silha JV, Gui Y, and Murphy LJ. Impaired glucose homeostasis in insulin-like growth factor-binding protein-3-transgenic mice. Am J Physiol Endocrinol Metab 283: E937-945, 2002.
46. Sparks LM, Xie H, Koza RA, Mynatt R, Hulver MW, Bray GA, and Smith SR. A High-Fat Diet Coordinately Downregulates Genes Required for Mitochondrial Oxidative Phosphorylation in Skeletal Muscle. Diabetes 54: 1926-1933, 2005.
47. Tuncman G, Hirosumi J, Solinas G, Chang L, Karin M, and Hotamisligil GS. Functional in vivo interactions between JNK1 and JNK2 isoforms in obesity and insulin resistance. PNAS 103: 10741-10746, 2006.
48. Turnbaugh PJ, Ley RE, Mahowald MA, Magrini V, Mardis ER, and Gordon JI. An obesity-associated gut microbiome with increased capacity for energy harvest. Nature 444: 1027, 2006.
49. Vega RB, Huss JM, and Kelly DP. The Coactivator PGC-1 Cooperates with Peroxisome Proliferator-Activated Receptor alpha in Transcriptional Control of Nuclear Genes Encoding Mitochondrial Fatty Acid Oxidation Enzymes. Mol Cell Biol 20: 1868-1876, 2000.
50. Wang L, Liu J, Saha P, Huang J, Chan L, Spiegelman B, and More DD. The orphan nuclear receptor SHP regulates PGC-1[alpha] expression and energy production in brown adipocytes. Cell Metabolism 2: 227, 2005.
51. Winzell MS, and Ahren B. The high-fat diet-fed mouse: a model for studying mechanisms and treatment of impaired glucose tolerance and type 2 diabetes. Diabetes 53 Suppl 3: S215-219, 2004.

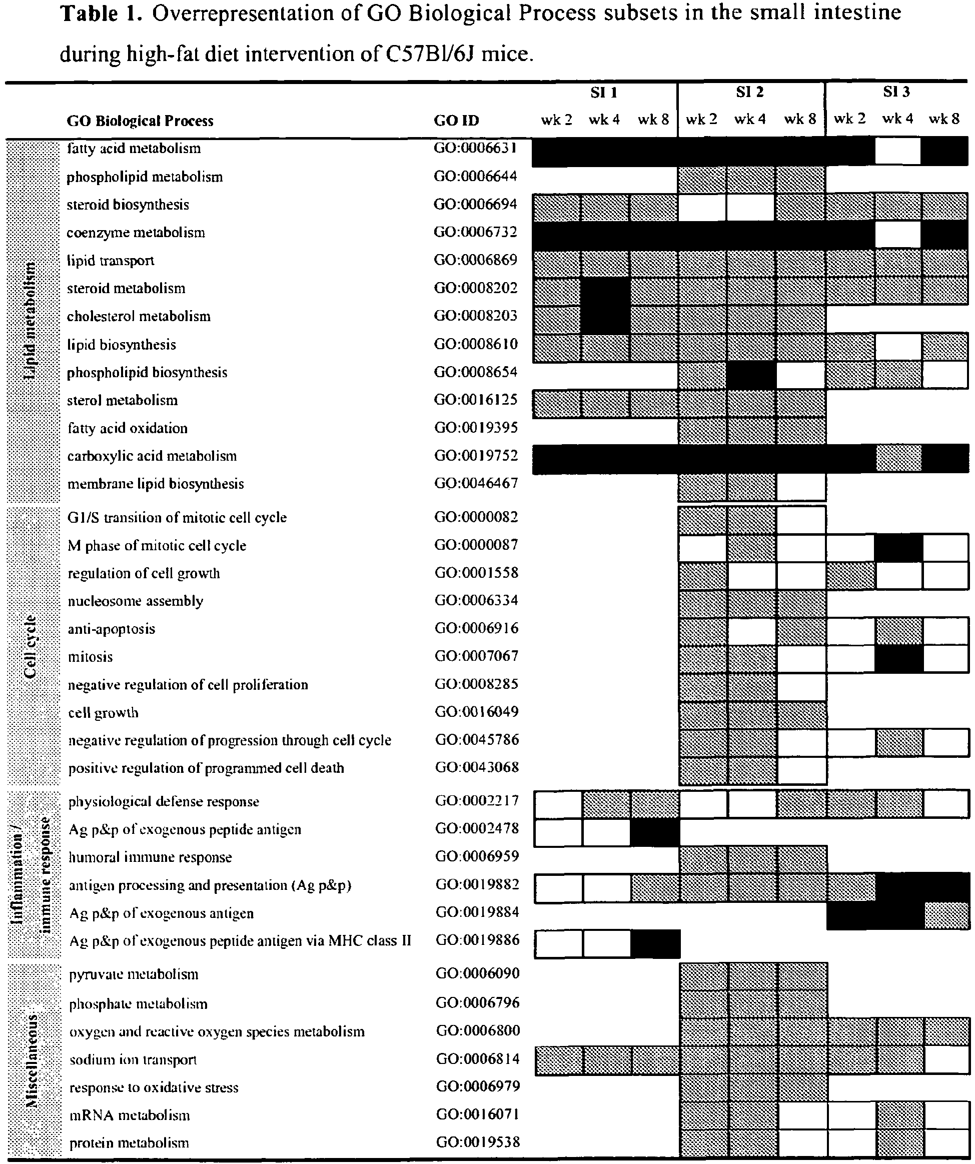

For the proximal (SI 1), middle (SI 2) and distal part of the small intestine (SI 3), GO Biological Process subsets with a FDR < 0.01 and a RawScore ≥ 10 in at least one week of diet intervention are included. Black boxes indicate 1.0E-31 < FDR < 1.0E-08; dark grey boxes indicate 1.0E-08 < FDR < 0.01; white boxes indicate FDR > 0.01, so not significant. An empty row indicates that this part of small intestine does meet the above mentioned selection criteria.

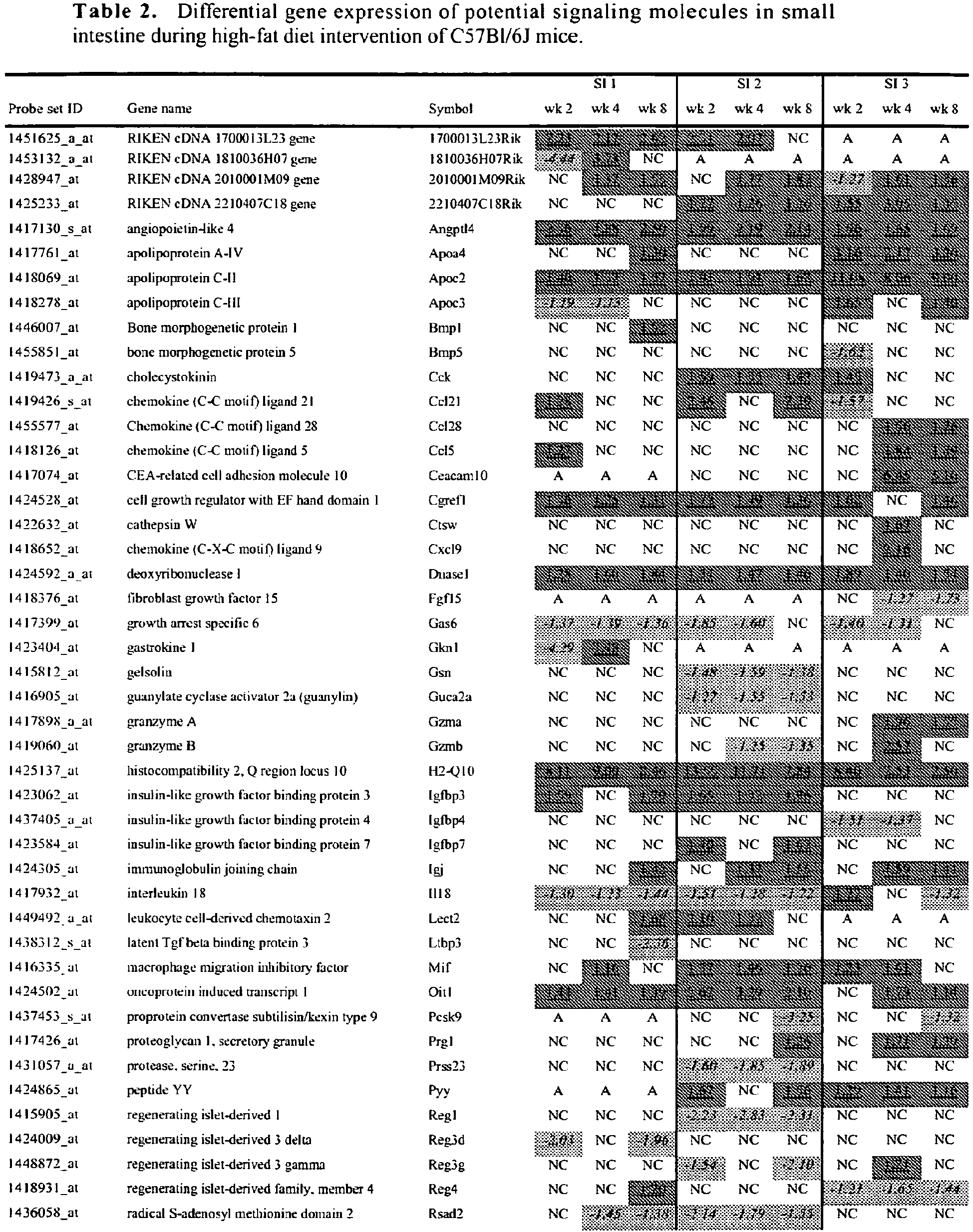

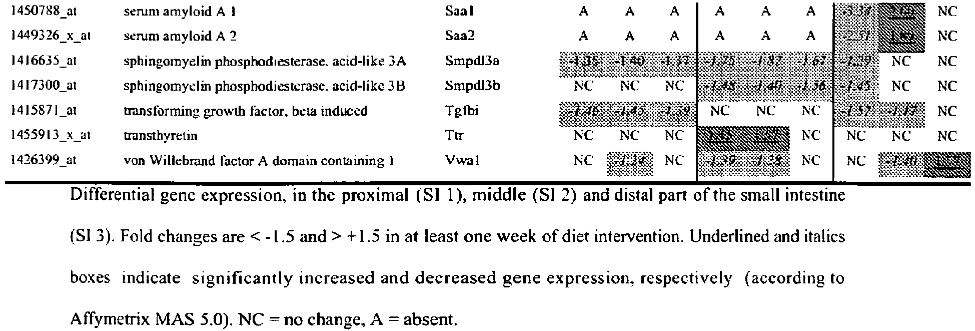

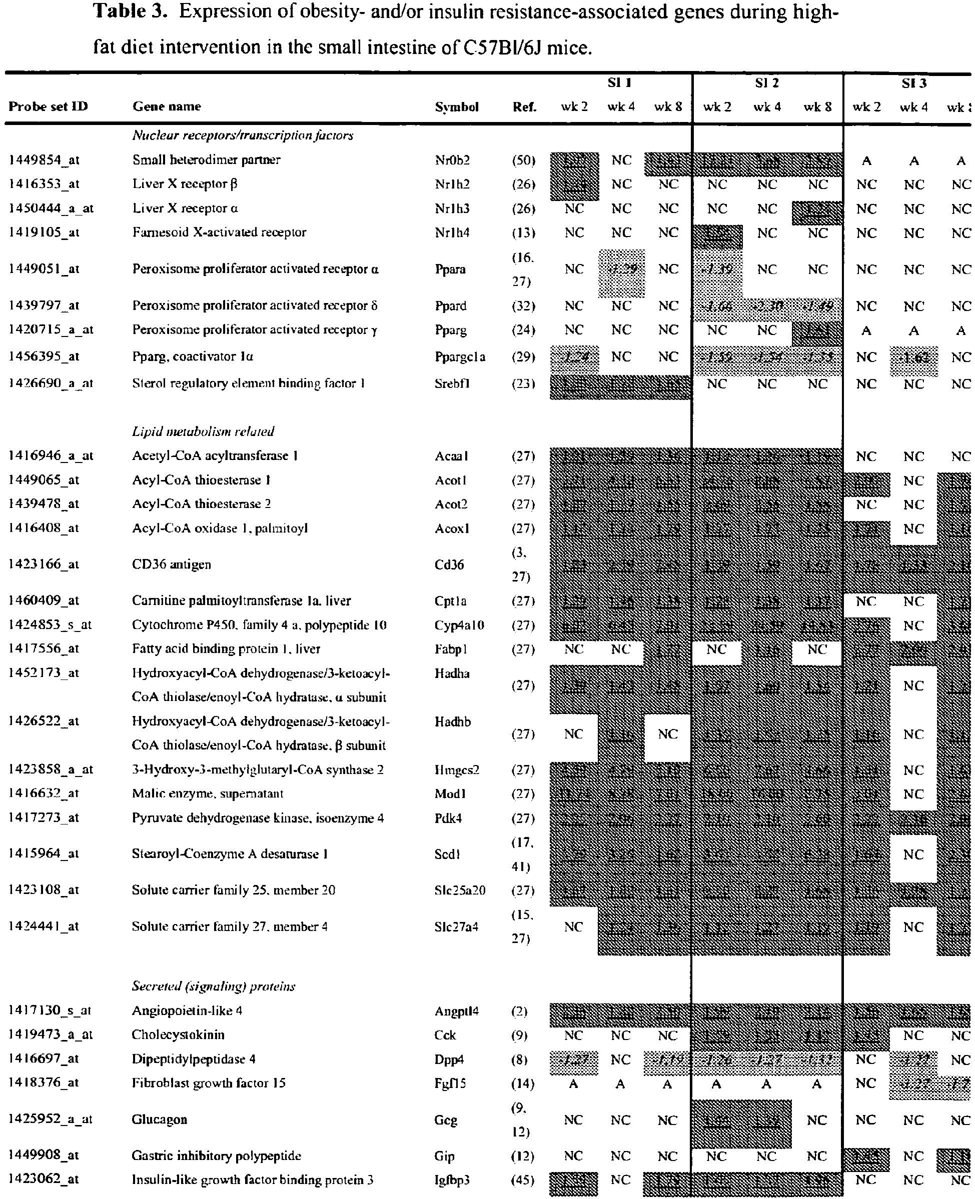

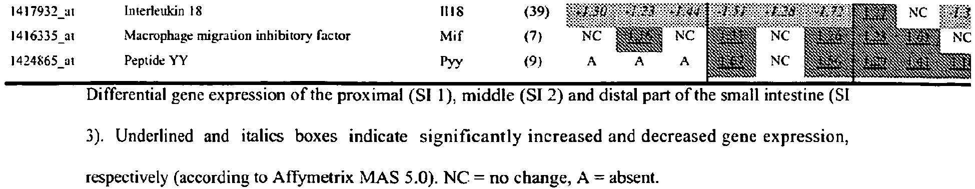

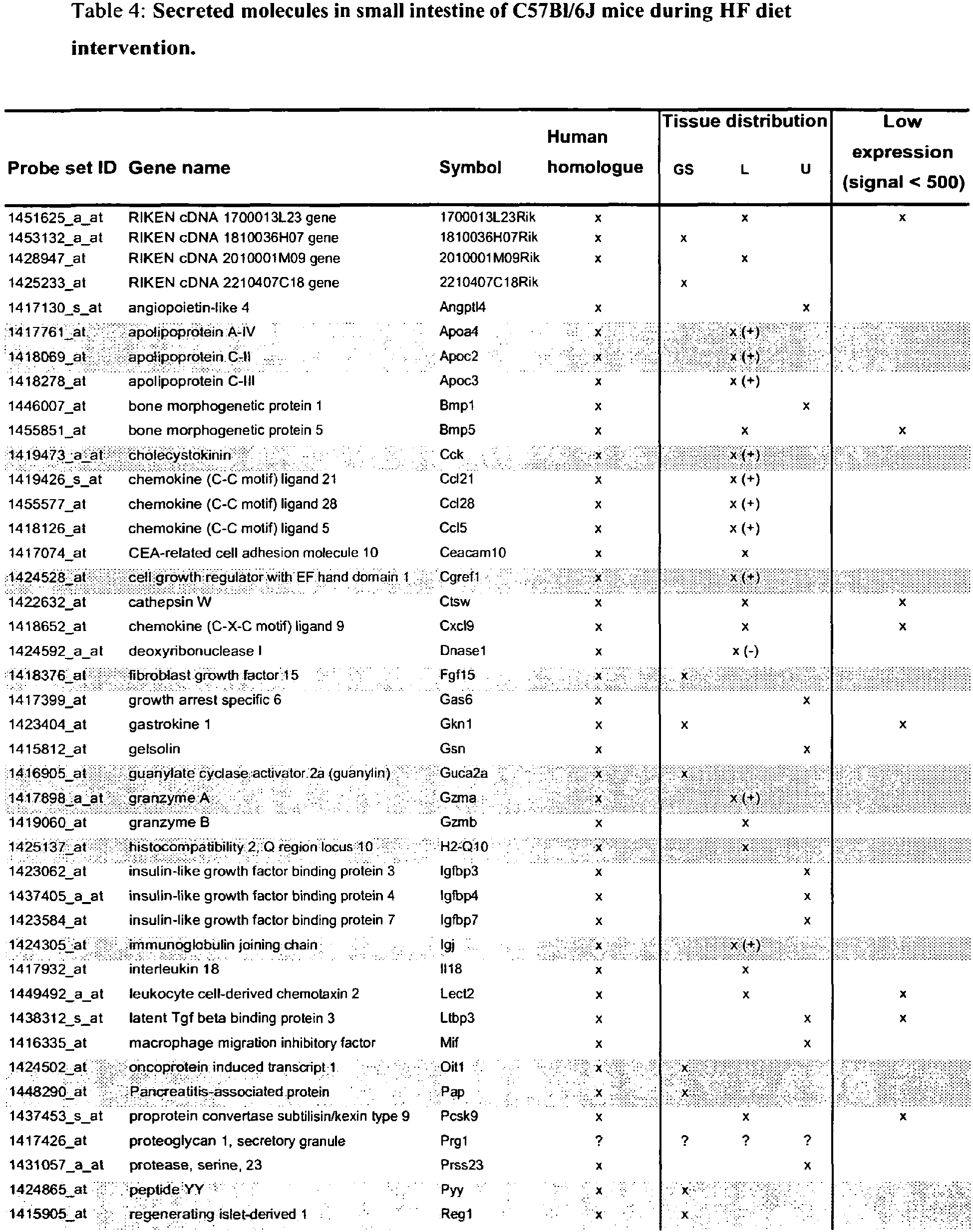

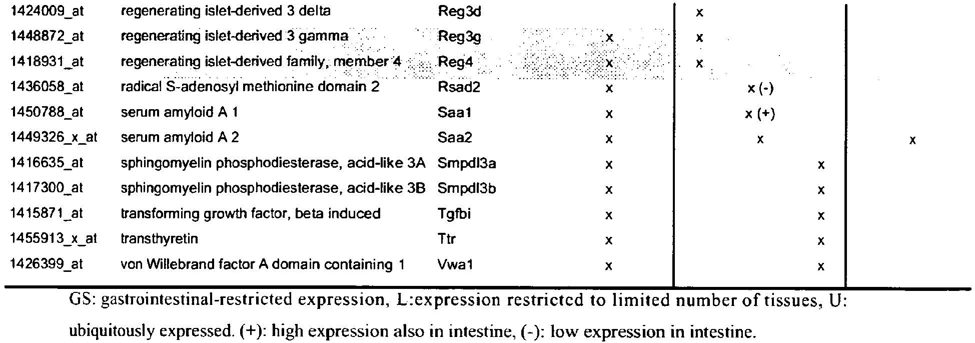

**Table 5**

| SEQ ID NO: cDNA | Symbol |
|---|---|
| (SEQ ID NO): protein | Gene name |
| 1(19) | Fam3 D or Oit 1 |
| | Oncoprotein induced transcript 1 |
| 2 (20) | Apoa4 |
| | Alipoprotein A-IV |
| 3(21) | Apoc2 |
| | Alipoprotein C-II |
| 4(22) | Cck |
| | Cholecystokinin |
| 5(23) | Cgrefl |
| | Cell growth regulator with EF hand |
| | Domain 1 |
| 6 (24) | Fgfl9 human homologue of Fgf15 |
| | Fibroblast growth factor 15 |
| 7 (25) | Guca2a |
| | Guanylate cyclase activator 2a (guanylin) |
| 8 (26) | Gzma |
| | Granzyme A |
| 9 (27) | HLA-G human homologue of H2-Q10 |
| | Histocompatibility 2, Q region locus 10 |
| 10 (28) | Igj |
| | Immunoglobulin joining chain |
| 11, 12 (29) | Reg3g human homologue of Pap |
| | Pancreatitis-associated protein |
| 13 (30) | Pyy |
| | Peptide YY |
| 14 (31) | Reg1a human homologue of Reg 1 |
| | Regenerating islet-derived 1 |
| 15, 16, 17 (32) | Reg3a human homologue of Reg 3g |
| | Regenerating islet-derived 3 gamma |
| 18 (33) | Reg 4 |
| | Regenerating islet-derived family, member 4 |

### Supplementary table S1. Diet composition

| Based on formula # | **Low fat (LF) diet** D12450B* | | **High fat (HF) diet** D12451* | |
|---|---|---|---|---|
| | **gm%** | ***kcal%*** | **gm%** | ***kcal%*** |
| Protein | 19 | 20 | 24 | 20 |
| Carbohydrate | 67 | 70 | 41 | 35 |
| Fat | 4 | 10 | 24 | 45 |

| **Ingredients** | **gm** | ***kcal*** | **gm** | ***kcal*** |
|---|---|---|---|---|
| Casein, lactic | 200 | 800 | 200 | 800 |
| L-Cystine | 3 | 12 | 3 | 12 |
| Corn Starch | 427.2 | *1709* | 72.8 | 291 |
| Maltodextrin | 100 | *400* | 100 | *400* |
| Sucrose | 172.8 | 691 | 172.8 | 691 |
| Cellulose, BW200 | 50 | 0 | 50 | 0 |
| Soybean Oil | 25 | 225 | 25 | 225 |
| Palm oil | 20 | *180* | 177.5 | 1598 |
| Mineral Mix S10026 | 10 | 0 | 10 | 0 |
| DiCalcium Phosphate | 13 | 0 | 13 | 0 |
| Calcium Carbonate | 5.5 | 0 | 5.5 | 0 |
| Potassium Citrate, 1 | | | | |
| H2O | 16.5 | 0 | 16.5 | 0 |
| Vitamin Mix V10001 | 10 | 40 | 10 | 40 |
| Choline Bitartrate | *2* | *0* | *2* | *0* |
| **Total** | 1055 | *4057* | 858.15 | *4057* |
| * Research Diets, Inc. (New Brunswick, NJ, USA) | | | | |

### Supplementary table S2. Primer sequences

| **Gene symbol** | **Forward primer** | **Reverse primer** |
|---|---|---|
| Abca1 | 5'-CCCAGAGCAAAAAGCGACTC-3' | 5'-ACCATCCATGCCTACAACAAAAGG-3' |
| Apoa4 | 5'-CAACAGGCTGAAGGCTACGAT-3' | 5'-CGATTTTTGCGGAGACCTTGG-3' |
| Ccnd1 | 5'-CAGAAGTGCGAAGAGGAGGTC-3' | 5'-TCATCTTAGAGGCCACGAACAT-3' |
| Cd36 | 5'-TCCAGCCAATGCCTTTGC-3' | 5'-TGGAGATTACTTTTCAGTGCAGAA-3' |
| Gsta3 | 5'-TAGAGATCGACGGGATGAAACT-3' | 5'-CAGATCCGCCACTCCTTCT-3' |
| Hmgcs2 | 5'-TGGTGGATGGGAAGCTGTCTA-3' | 5'-TTCTTGCGGTAGGCTGCATAG-3' |
| II18 | 5'-GACTCTTGCGTCAACTTCAAGG-3' | 5'-CAGGCTGTCTTTTGTCAACGA-3' |
| Mttp | 5'-ATACAAGCTCACGTACTCCACT-3' | 5'-TCCACAGTAACACAACGTCCA-3' |
| Scarb1 | 5'-TTTGGAGTGGTAGTAAAAAGGG-3' | 5'-TGACATCAGGGACTCAGAGTAG-3' |
| Scd1 | 5'-CCGGAGACCCTTAGATCGA-3' | 5'-TAGCCTGTAAAAGATTTCTGCAAACC-3' |
| Slc25a20 | 5'-CCGAAACCCATCAGTCCGTTTAA-3' | 5'-ACATAGGTGGCTGTCCAGACAA-3' |

### Supplementary table 3. Genes showing a consistent differential expression in the small intestine of C57Bl/6J mice in all weeks of diet intervention.

| | | | | **Fold change** | | |
|---|---|---|---|---|---|---|
| | **Probe set ID** | **Gene name** | **Symbol** | **wk 2** | **wk 4** | **wk 8** |
| ***SI 1*** | 1416632_at | malic enzyme, supernatant | Mod1 | 11.24 | 8.28 | 7.01 |
| | 1425137_a_at | histocompatibility 2, Q region locus 10 | H2-010 | 8.11 | 9.00 | 8.46 |
| | 1436169_at | RIKEN cDNA C730029A08 gene | C730029A08Rik | 7.26 | 3.27 | 3.56 |
| | 1424853_s_at | cytochrome P450, family 4, subfamily a, polypeptide 10 | Gyp4a10 | 6.02 | 6.45 | 7.01 |
| | 1448700_at | G0/G1 switch gene 2 | G0s2 | 5.43 | 5.35 | 9.25 |
| | 1423858_a_at | 3-hydroxy-3-methylglutaryl-Coenzyme A synthase 2 | Hmgcs2 | 4.59 | 4.79 | 5.10 |
| | 1449065_at | acyl-CoA thioesterase 1 | Acot1 | 3.71 | 4.38 | 6.63 |
| | 1419622_at | UDP glucuronosyltransferase 2 family, polypeptide B5 | Ugt2b5 | 3.41 | 2.87 | 3.66 |
| | 1415964_at | stearoyl-Coenzyme A desaturase 1 | Scd1 | 3.29 | 3.25 | 1.68 |
| | 1418538_at | KDEL endoplasmic reticulum protein retention receptor | Kdelr3 | 2.95 | 4.00 | 2.99 |
| | 1424167_a_at | phosphomannomutase 1 | Pmm1 | 2.35 | 2.13 | 3.29 |
| | 1433626_at | phospholipid scramblase 4 | Piscr4 | 2.31 | 2.75 | 3.76 |
| | 1429286_at | RIKEN cDNA 1190003M12 gene | 1190003M12Rik | 1.42 | 1.79 | 5.82 |
| | 1449907_at | beta-carotene 15,15'-monooxygenase | Bcmo1 | -9.71 | -14.52 | -13.36 |
| | 1418787_at | mannose binding lectin (C) | Mbl2 | -5.50 | -16.68 | -8.11 |
| | 1421840_at | ATP-binding cassette, sub-family A, member 1 | Abca1 | -3.18 | -2.81 | -2.31 |
| | 1417651_at | cytochrome P450, family 2, subfamily c, polypeptide 29 | Cyp2c29 | -2.60 | -3.16 | -2.48 |
| | 1435370_a_at | carboxylesterase 3 | Ces3 | -2.33 | -3.81 | -2.99 |
| ***SI 2*** | 1449065_at | acyl-CoA thioesterase 1 | Acot1 | 24.76 | 8.88 | 6.87 |
| | 1424853_s_at | cytochrome P450, family 4, subfamily a, polypeptide 10 | Gyp4a10 | 23.59 | 24.59 | 14 83 |
| | 1416632_at | malic enzyme, supernatant | Mod1 | 18.00 | 16.00 | 8.75 |
| | 1425137_a_at | histocompatibility 2, Q region locus 10 | H2-Q10 | 13.55 | 11.71 | 7.84 |
| | 1449854_at | nuclear receptor subfamily 0, group B, member 2 | Nr0b2 | 12.21 | 3.68 | 2.89 |
| | 1424266_s_at | expressed sequence AU018778 | AU018778 | 677 | 6.54 | 4.59 |
| | 1423858_a_at | 3-hydroxy-3-methylglutaryl-Coenzyme A synthase 2 | Hmgcs2 | 650 | 7.67 | 4.66 |
| | 1448700_at | G0/G1 switch gene 2 | G0s2 | 628 | 7.62 | 8.94 |
| | 1431688_at | hypothetical LOC73899 | LOC73899 | 5.94 | 2.01 | 4.56 |
| | 1418538_at | KDEL endoplasmic reticulum protein retention receptor | Kdelr3 | 558 | 4.59 | 4.20 |
| | 1421040_a_at | glutathione S-transferase, alpha 2 (Yc2) | Gsta2 | 5.10 | 3.03 | 2.57 |
| | 1419692_a_at | leukotriene C4 synthase | Ltc4s | 4.82 | 3.76 | 3.76 |
| | 1419618_at | butyrobetaine (gamma), 2-oxoglutarate dioxygenase 1 | Bbox1 | 4.76 | 5.46 | 3.89 |
| | 1427347_s_at | tubulin, beta 2 | Tubb2 | 4 69 | 4.17 | 2.69 |
| | 1417812_a_at | laminin, beta 3 | Lamb3 | 4 66 | 4.06 | 3.53 |
| | 1419622_at | UDP glucuronosyltransferase 2 family, polypeptide B5 | Ugt2b5 | 4.35 | 4.20 | 4.00 |
| | 1456558_s_at | expressed sequence C87977 | C87977 | 4.35 | 2.91 | 2.71 |
| | 1432790_at | RIKEN cDNA 9030218A15 gene | 9030218A15Rik | 417 | 2.10 | 1.65 |
| | 1423436_at | glutathione S-transferase, alpha 3 | Gsta3 | 4.08 | 3.46 | 3.56 |
| | 1417415_at | solute carrier family 6, member 3 | Slc6a3 | 3.68 | 5.86 | 5.17 |
| | 1418848_at | aquaporin 7 | Aqp7 | 3.66 | 3.39 | 4.32 |
| | 1415964_at | stearoyl-Coenzyme A desaturase 1 | Scd1 | 3.63 | 3.58 | 6.28 |
| | 1430780_a_at | phosphomannomutase 1 | Pmm1 | 3.63 | 4.03 | 2.11 |
| | 1452277_at | RIKEN cDNA 6330406P08 gene | 6330406P08Rik | 3.63 | 3.05 | 2.48 |
| | 1429298_at | dimethylarginine dimethylaminohydrolase 1 | Ddah1 | 3.48 | 2.45 | 1.53 |
| | 1459030_at | - | - | 3.39 | 3.51 | 3.39 |
| | 1424962_at | transmembrane 4 superfamily member 4 | Tm4sf4 | 3.36 | 3.32 | 2.51 |
| | 1420673_a_at | acyl-Coenzyme A oxidase 2, branched chain | Acox2 | 3.01 | 2.87 | 2.33 |
| | 1426452_a_at | RAB30, member RAS oncogene family | Rsb30 | 3.01 | 2.91 | 2.01 |
| | 1448777_at | minichromosome maintenance deficient 2 | Mcm2 | 2.71 | 3.05 | 2.89 |
| | 1433626_at | phospholipid scramblase 4 | Plscr4 | 2.71 | 4.08 | 5.17 |
| | 1424502_at | oncoprotein induced transcript 1 | Oit1 | 2.62 | 3.29 | 2.10 |
| | 1459059_at | RIKEN cDNA 2010308F09 gene | 2010308F09Rik | 2.11 | 5.39 | 1.28 |
| | 1449907_at | beta-carotene 15,15'-monooxygenase | Bcmo1 | -34.54 | -30.91 | -19.84 |
| | 1418787_at | mannose binding lectin (C) | Mbl2 | -14.32 | -17.88 | -8.00 |
| | 1424265_at | N-acetylneuraminate pyruvate lyase | Npl | -5.66 | -6.77 | -6.59 |
| | 1416050_a_at | scavenger receptor dass B, member 1 | Scarb1 | -4.82 | -4.17 | -3.39 |
| | 1450167_at | RAB37, member of RAS oncogene family | Rab37 | -4.29 | -3.16 | -1.97 |
| | 1450392_at | ATP-binding cassette, sub-family A, member 1 | Abca1 | -3.41 | -2.60 | -1.92 |
| | 1434736_at | hepatic leukemia factor | Hlf | -3.27 | -3.14 | -2.62 |
| | 1418382_at | adenomatosis polyposis coli down-regulated 1 | Apcdd1 | -2.95 | -3.25 | -2.22 |
| | 1436021_at | RIKEN cDNA A930031 D07 gene | A930031 D07Rik | -2.79 | -2.45 | -3.51 |
| | 1416432_at | 6-phosphofructo-2-kinase/fructose-2,6-biphosphatase 3 | Pfkfb3 | -2.41 | -3.05 | -3.27 |
| | 1418979_at | RIKEN cDNA 9030611 N15 gene | 9030611N15Rik | -2.36 | -2.35 | -2.17 |
| | 1438610_a_at | Crystallin, zeta | Cryz | -2.36 | -4.41 | -1.58 |
| | 1435370_a_at | carboxylesterase 3 | Ces3 | -2.11 | -3.01 | -2.46 |
| ***SI 3*** | 1418069_at | apolipoprotein C-II | Apoc2 | 11.08 | 8.06 | 9.00 |
| | 1425137_a_at | histocompatibility 2, Q region locus 10 | H2-Q10 | 8.40 | 2.53 | 2.50 |
| | 1422846_at | retinol binding protein 2 | Rbp2 | 3.43 | 2.28 | 3.10 |
| | 1417761_at | apolipoprotein A-IV | Apoa4 | 3.16 | 2.17 | 3.20 |
| | 1425233_at | RIKEN cDNA 2210407C18 gene | 2210407C18Rik | 1.55 | 3.05 | 1.35 |
| | 1449907_at | beta-carotene 15,15'-monooxygenase | Bcmo1 | -3.63 | -3.39 | -2.30 |
| | 1424265_at | N-acelylneuraminate pyruvate lyase | Npl | -2.38 | -3.46 | -2.17 |
| | 1418174_at | D site albumin promoter binding protein | Dbp | -2.08 | -3.43 | -1.89 |
| Consistent differential gene expression in the proximal (SI 1), middle (SI 2) and distal part of the small intestine (SI 3). Fold changes are < -3.0 and > +3.0 in at least one week of diet intervention. | | | | | | |

## Claims

1. A method for diagnosing pre-symptomatic metabolic syndrome in a subject, the method comprising the steps of:
(a) determining *ex vivo* the expression level of a gene represented by a nucleotide sequence consisting of SEQ ID NO: 1 in a subject; and,
(b) comparing the expression level of said gene as defined in (a) with a reference value for said expression level, the reference value preferably being the average value for said expression level in a control subject.

2. A method according to claim 1, wherein a pre-symptomatic metabolic syndrome is diagnosed when the comparison leads to the finding of a detectable expression level or an increase of the expression level of said gene.

3. A method according to claim 1 or 2, wherein the expression level of said gene is determined by directly quantifying the amount of encoded polypeptide and/or indirectly by quantifying the amount of a corresponding nucleotide molecule.

4. A method according to claim 3, wherein the sample is a fluid obtained from the subject, preferably wherein the fluid is selected from: plasma, faeces, urine, blood, or saliva.

5. A method according to any one of claims 1 to 4, wherein step (a) further comprises determining the expression level of a gene represented by a nucleotide sequence selected from the group consisting of SEQ ID NO:2-18 in a subject, and step (b) further comprises comparing the expression level of said genes as defined in (a) with a reference value for said expression level, the reference value preferably being the average value for said expression level in a control subject.

6. A method for identification of a substance capable of preventing, treating and/or delaying the progression of metabolic syndrome in a subject, the method comprising the steps of:
(a) providing a test cell population or a non-human test animal capable of expressing a gene represented by a sequence as identified in claim 1;
(b) contacting the test cell population or the non-human test animal with the substance;
(c) determining the expression level of the gene encoding a polypeptide or the activity or steady state level of the encoded polypeptide in the test cell population or in the non-human test animal contacted with the substance;
(d) comparing the expression, activity or steady state level determined in (c) with the expression, activity or steady state level of the gene or of the polypeptide in a test cell population or in a non-human test animal that is not contacted with the substance; and,
(e) identifying a substance that produces a difference in expression level, activity or steady state level of the gene or the polypeptide, between the test cell population or non-human test animal that is contacted with the substance and the test cell population or non-human test animal that is not contacted with the substance.

7. A method according to claim 6, whereby the difference identified in step (d) produced by the substance is a decrease of the expression level of said gene.

8. A method according to claim 6 or 7, wherein the expression level of said gene is determined by directly quantifying the amount of encoded polypeptide and/or indirectly by quantifying the amount of a gene encoding said polypeptide.

9. A method according to any one of claims 6 to 8, whereby the test cell population comprises mammalian, preferably human cells or the non-human test animal is a C57BL/6J mouse.

10. A method according to any one of claims 6 to 9, whereby the test cell population comprises colon carcinoma cell lines LS174T and LOVO.

## Patentansprüche

1. Verfahren zum Diagnostizieren von präsymptomatischem metabolischem Syndrom bei einem Probanden, wobei das Verfahren die Schritte umfasst:
(a) den Expressionsspiegel eines Gens, das durch eine aus SEQ ID NO: 1 bestehende Nucleotidsequenz dargestellt wird, bei einem Probanden *ex vivo* zu bestimmen, und
(b) den Expressionsspiegel des wie in (a) definierten Gens mit einem Referenzwert für den Expressionsspiegel zu vergleichen, wobei es sich bei dem Referenzwert vorzugsweise um den Mittelwert für den Expressionsspiegel bei einem Kontrollprobanden handelt.

2. Verfahren gemäß Anspruch 1, wobei ein präsymptomatisches metabolisches Syndrom diagnostiziert wird, wenn der Vergleich zur Feststellung eines nachweisbaren Expressionsspiegels oder einer Erhöhung des Expressionsspiegels des Gens führt.

3. Verfahren gemäß Anspruch 1 oder 2, wobei der Expressionsspiegel des Gens durch direktes Quantifizieren der Menge des kodierten Polypeptids und/oder indirekt durch Quantifizieren der Menge eines entsprechenden Nucleotidmoleküls bestimmt wird.

4. Verfahren gemäß Anspruch 3, wobei es sich bei der Probe um eine von dem Probanden erhaltene Flüssigkeit handelt, wobei die Flüssigkeit vorzugsweise aus Plasma, Fäzes, Urin, Blut oder Speichel ausgewählt ist.

5. Verfahren gemäß einem der Ansprüche 1 bis 4, wobei Schritt (a) ferner das Bestimmen des Expressionsspiegels eines Gens bei einem Probanden umfasst, das durch eine aus der Gruppe, bestehend aus SEQ ID NO: 2-18, ausgewählte Nucleotidsequenz dargestellt wird, und Schritt (b) ferner das Vergleichen des Expressionsspiegels des wie in (a) definierten Gens mit einem Referenzwert für den Expressionsspiegel umfasst, wobei es sich bei dem Referenzwert vorzugsweise um den Mittelwert für den Expressionsspiegel bei einem Kontrollprobanden handelt.

6. Verfahren zum Identifizieren einer zum Vorbeugen, Behandeln und/oder Verzögern des Fortschreitens von metabolischem Syndrom bei einem Probanden fähigen Substanz, wobei das Verfahren die Schritte umfasst:
(a) Bereitstellen einer Test-Zellpopulation oder eines nicht menschlichen Test-Tieres, welche bzw. welches zum Exprimieren eines Gens, das durch eine wie in Anspruch 1 identifizierte Sequenz dargestellt ist, fähig ist;
(b) In-Kontakt-Bringen der Test-Zellpopulation oder des nicht menschlichen Test-Tieres mit der Substanz;
(c) Bestimmen des Expressionsspiegels des Gens, welches ein Polypeptid kodiert, oder der Aktivität oder des Steady-State-Spiegels des kodierten Polypeptids in der Test-Zellpopulation oder in dem nicht menschlichen Test-Tier, welche bzw. welches mit der Substanz in Kontakt gebracht wurde;
(d) Vergleichen der in (c) bestimmten Expression, Aktivität oder des Steady-State-Spiegels mit der Expression, Aktivität oder dem Steady-State-Spiegel des Gens oder des Polypeptids in einer Test-Zellpopulation oder in einem nicht menschlichen Test-Tier, welche bzw. welches nicht mit der Substanz in Kontakt gebracht wurde, und
(e) Identifizieren einer Substanz, die einen Unterschied im Expressionsspiegel, in der Aktivität oder im Steady-State-Spiegel des Gens oder des Polypeptids zwischen der Test-Zellpopulation oder dem nicht menschlichen Test-Tier, welche bzw. welches mit der Substanz in Kontakt gebracht wurde, und der Test-Zellpopulation oder dem nicht menschlichen Test-Tier, welche bzw. welches nicht mit der Substanz in Kontakt gebracht wurde, produziert.

7. Verfahren gemäß Anspruch 6, wobei es sich bei dem in Schritt (d) identifizierten, durch die Substanz produzierten Unterschied um eine Senkung des Expressionsspiegels des Gens handelt.

8. Verfahren gemäß Anspruch 6 oder 7, wobei der Expressionsspiegel des Gens durch direktes Quantifizieren der Menge des kodierten Polypeptids und/oder indirekt durch Quantifizieren der Menge eines das Polypeptid kodierenden Gens bestimmt wird.

9. Verfahren gemäß einem der Ansprüche 6 bis 8, wobei die Test-Zellpopulation Säuger-, vorzugsweise menschliche Zellen umfasst oder es sich bei dem nicht menschlichen Test-Tier um eine C57BL/6J-Maus handelt.

10. Verfahren gemäß einem der Ansprüche 6 bis 9, wobei die Test-Zellpopulation die Kolonkarzinomzelllinien LS174T und LOVO umfasst.

## Revendications

1. Procédé pour diagnostiquer un syndrome métabolique présymptomatique chez un sujet, le procédé comprenant les étapes consistant à :
(a) déterminer *ex vivo* le niveau d'expression d'un gène représenté par une séquence nucléotidique constituée par SEQ ID N° : 1 chez un sujet ; et
(b) comparer le niveau d'expression dudit gène tel que défini en (a) avec une valeur de référence dudit niveau d'expression, la valeur de référence étant de préférence la valeur moyenne dudit niveau d'expression chez un sujet témoin.

2. Procédé selon la revendication 1, dans lequel un syndrome métabolique présymptomatique est diagnostiqué lorsque la comparaison conduit à la découverte d'un niveau d'expression détectable ou d'une augmentation du niveau d'expression dudit gène.

3. Procédé selon la revendication 1 ou 2, dans lequel le niveau d'expression dudit gène est déterminé en quantifiant directement la quantité de polypeptide codé et/ou en quantifiant indirectement la quantité d'une molécule de nucléotide correspondante.

4. Procédé selon la revendication 3, dans lequel l'échantillon est un fluide obtenu auprès du sujet, de préférence dans lequel le fluide est sélectionné parmi : le plasma, les matières fécales, l'urine, le sang ou la salive.

5. Procédé selon l'une quelconque des revendications 1 à 4, dans lequel l'étape (a) comprend en outre la détermination du niveau d'expression d'un gène représenté par une séquence nucléotidique sélectionnée dans le groupe constitué par SEQ ID N° : 2 à 18 chez un sujet, et l'étape (b) comprend en outre la comparaison du niveau d'expression desdits gènes tel que défini en (a) avec une valeur de référence dudit niveau d'expression, la valeur de référence étant de préférence la valeur moyenne dudit niveau d'expression chez un sujet témoin.

6. Procédé d'identification d'une substance capable de prévenir, traiter et/ou différer la progression d'un syndrome métabolique chez un sujet, le procédé comprenant les étapes consistant à :
(a) fournir une population de cellules test ou un animal test non humain capable d'exprimer un gène représenté par une séquence telle qu'identifiée dans la revendication 1 ;
(b) mettre en contact la population de cellules test ou l'animal test non humain avec la substance ;
(c) déterminer le niveau d'expression du gène codant pour un polypeptide ou le niveau d'activité ou stabilisé du polypeptide codé dans la population de cellules test ou chez l'animal test non humain en contact avec la substance ;
(d) comparer le niveau d'expression, d'activité ou stabilisé tel que déterminé en (c) avec le niveau d'expression, d'activité ou stabilisé du gène ou du polypeptide dans une population de cellules test ou chez un animal test non humain qui n'est pas en contact avec la substance ; et
(e) identifier une substance qui produit une différence de niveau d'expression, de niveau d'activité ou stabilisé du gène ou du polypeptide, entre la population de cellules test ou un animal test non humain en contact avec la substance et la population de cellules test ou un animal test non humain qui n'est pas en contact avec la substance.

7. Procédé selon la revendication 6, dans lequel la différence identifiée à l'étape (d) produite par la substance est une diminution du niveau d'expression dudit gène.

8. Procédé selon la revendication 6 ou 7, dans lequel le niveau d'expression dudit gène est déterminé en quantifiant directement la quantité de polypeptide codé et/ou en quantifiant indirectement la quantité d'un gène codant pour ledit polypeptide.

9. Procédé selon l'une quelconque des revendications 6 à 8, dans lequel la population de cellules test comprend des cellules de mammifères, de préférence des cellules humaines ou l'animal test non humain est une souris C57BL/6J.

10. Procédé selon l'une quelconque des revendications 6 à 9, dans lequel la population de cellules test comprend des lignées cellulaires de carcinome du côlon LS174T et LOVO.


### SEQUENCE LISTING

<110> Stichting Top Institute Food and Nutrition
<120> Diagnostic of presymptomatic metabolic syndrome
<130> P6018342PCT
<150> US 61/038,433
   <151> 2008-03-21
<150> EP 08156294.4
   <151> 2008-05-15
<160> 59
<170> PatentIn version 3.3
<210> 1
   <211> 1322
   <212> DNA
   <213> homo sapiens
<400> 1
   tcctcaaagg aggggcagag cctgcgcagg gcaggagcag ctggcccact ggcggcccgc      60
   aacactccgt ctcaccctct gggcccactg catctagagg agggccgtct gtgaggccac     120
   tacccctcca gcaactggga ggtgggactg tcagaagctg gcccagggtg gtggtcagct    180
   gggtcaggga cctacggcac ctgctggacc acctcgcctt ctccatcgaa gcagggaagt     240
   gggagcctcg agccctcggg tggaagctga ccccaagcca cccttcacct ggacaggatg     300
   agagtgtcag gtgtgcttcg cctcctggcc ctcatctttg ccatagtcac gacatggatg     360
   tttattcgaa gctacatgag cttcagcatg aaaaccatcc gtctgccacg ctggctggca     420
   gcctcgccca ccaaggagat ccaggttaaa aagtacaagt gtggcctcat caagccctgc     480
   ccagccaact actttgcgtt taaaatctgc agtggggccg ccaacgtcgt gggccctact     540
   atgtgctttg aagaccgcat gatcatgagt cctgtgaaaa acaatgtggg cagaggccta     600
   aacatcgccc tggtgaatgg aaccacggga gctgtgctgg gacagaaggc atttgacatg     660
   tactctggag atgttatgca cctagtgaaa ttccttaaag aaattccggg gggtgcactg     720
   gtgctggtgg cctcctacga cgatccaggg accaaaatga acgatgaaag caggaaactc     780
   ttctctgact tggggagttc ctacgcaaaa caactgggct tccgggacag ctgggtcttc     840
   ataggagcca aagacctcag gggtaaaagc ccctttgagc agttcttaaa gaacagccca     900
   gacacaaaca aatacgaggg atggccagag ctgctggaga tggagggctg catgcccccg     960
   aagccatttt agggtggctg tggctcttcc tcagccaggg gcctgaagaa gctcctgcct    1020
   gacttaggag tcagagcccg gcaggggctg aggaggagga gcagggggtg ctgcgtggaa    1080
   ggtgctgcag gtccttgcac gctgtgtcgc gcctctcctc ctcggaaaca gaaccctccc    1140
   acagcacatc ctacccggaa gaccagcctc agagggtcct tctggaacca gctgtctgtg    1200
   gagagaatgg ggtgctttcg tcagggactg ctgacggctg gtcctgagga aggacaaact    1260
   gcccagactt gagcccaatt aaattttatt tttgctggtt ttgaatgaaa aaaaaaaaaa    1320
   aa                                                                   1322
<210> 2
   <211> 1460
   <212> DNA
   <213> homo sapiens
<400> 2
   tgcagcgcag gtgagctctc ctgaggacct ctctgtcagc tcccctgatt gtagggagga 60
   tccagtgtgg caagaaactc ctccagccca gcaagcagct caggatgttc ctgaaggccg 120
   tggtcctgac cctggccctg gtggctgtcg ccggagccag ggctgaggtc agtgctgacc 180
   aggtggccac ggtgatgtgg gactacttca gccagctgag caacaatgcc aaggaggccg 240
   tggaacatct ccagaaatct gaactcaccc agcaactcaa tgccctcttc caggacaaac 300
   ttggagaagt gaacacttac gcaggtgacc tgcagaagaa gctggtgccc tttgccaccg 360
   agctgcatga acgcctggcc aaggactcgg agaaactgaa ggaggagatt gggaaggagc 420
   tggaggagct gagggcccgg ctgctgcccc atgccaatga ggtgagccag aagatcgggg 480
   acaacctgcg agagcttcag cagcgcctgg agccctacgc ggaccagctg cgcacccagg 540
   tcagcacgca ggccgagcag ctgcggcgcc agctgacccc ctacgcacag cgcatggaga 600
   gagtgctgcg ggagaacgcc gacagcctgc aggcctcgct gaggccccac gccgacgagc 660
   tcaaggccaa gatcgaccag aacgtggagg agctcaaggg acgccttacg ccctacgctg 720
   acgaattcaa agtcaagatt gaccagaccg tggaggagct gcgccgcagc ctggctccct 780
   atgctcagga cacgcaggag aagctcaacc accagcttga gggcctgacc ttccagatga 840
   agaagaacgc cgaggagctc aaggccagga tctcggccag tgccgaggag ctgcggcaga 900
   ggctggcgcc cttggccgag gacgtgcgtg gcaacctgag ggcaacaccc gaggggctgc 960
   agaagtcact ggcagagctg ggtgggcacc tggaccagca ggtggaggag ttccgacgcc 1020
   gggtggagcc ctacggggaa aacttcaaca aagccctggt gcagcagatg gaacagctca 1080
   ggcagaaact gggcccccat gcgggggacg tggaaggcca cttgagcttc ctggagaagg 1140
   acctgaggga caaggtcaac tccttcttca gcaccttcaa ggagaaagag agccaggaca 1200
   agactctctc cctccctgag ctggagcaac agcaggaaca gcagcaggag cagcagcagg 1260
   agcaggtgca gatgctggcc cctttggaga gctgagctgc ccctggtgca ctggccccac 1320
   cctcgtggac acctgccctg ccctgccacc tgtctgtctg tctgtcccaa agaagttctg 1380
   gtatgaactt gaggacacat gtccagtggg aggtgagacc acctctcaat attcaataaa 1440
   gctgctgaga atctagcctc 1460
<210> 3
   <211> 753
   <212> DNA
   <213> homo sapiens
<400> 3
   gttgtggctg tggagcggaa gtgggtctca accactataa atcctctctg tgcccgtccg 60
   gagctggtga ggacagcctg ccagagtctg gtctctggac actatgggca cacgactcct 120
   cccagctctg tttcttgtcc tcctggtatt gggatttgag gtccagggga cccaacagcc 180
   ccagcaagat gagatgccta gcccgacctt cctcacccag gtgaaggaat ctctctccag 240
   ttactgggag tcagcaaaga cagccgccca gaacctgtac gagaagacat acctgcccgc 300
   tgtagatgag aaactcaggg acttgtacag caaaagcaca gcagccatga gcacttacac 360
   aggcattttt actgaccaag ttctttctgt gctgaaggga gaggagtaac agccagaccc 420
   cccatcagtg gacaagggga gagtccccta ctcccctgat cccccaggtt cagactgagc 480
   tcccccttcc cagtagctct tgcatcctcc tcccaactct agcctgaatt cttttcaata 540
   aaaaatacaa ttcaagttgc ttctcatgga tggcactgct tttctgagga ctcaagggcc 600
   aagatggagg ggctgactca gtccagccaa catttaatga gcacctactt tatgtatgga 660
   gctctaaccc atgggtccat gggaataaag cagtgaatag taacaataaa taatcgtaac 720
   aaaaaaaaaa aaaaaaaaaa aaaaaaaaaa aaa 753
<210> 4
   <211> 851
   <212> DNA
   <213> homo sapiens
<400> 4
   cacttcaacc ggttgtcgcc ccagtggccg ccctctgagc acgtgttact gccagtctgc 60
   gtcagcgttg ggtaaataca tgactggccg acgccgccgg gcggggctat ttaagagaca 120
   gccgcccgct ggtcctccct gaacttggct cagctgccgg gctgctccgg ttggaaacgc 180
   caagccagct gcgtcctaat ccaaaagcca tgaacagcgg cgtgtgcctg tgcgtgctga 240
   tggcggtact ggcggctggc gccctgacgc agccggtgcc tcccgcagat cccgcgggct 300
   ccgggctgca gcgggcagag gaggcgcccc gtaggcagct gagggtatcg cagagaacgg 360
   atggcgagtc ccgagcgcac ctgggcgccc tgctggcaag atacatccag caggcccgga 420
   aagctccttc tggacgaatg tccatcgtta agaacctgca gaacctggac cccagccaca 480
   ggataagtga ccgggactac atgggctgga tggattttgg ccgtcgcagt gccgaggagt 540
   atgagtaccc ctcctagagg acccagccgc catcagccca acgggaagca acctcccaac 600
   ccagaggagg cagaataaga aaacaatcac actcataact cattgtctgt ggagtttgac 660
   attgtatgta tctatttatt aagttctcaa tgtgaaaaat gtgtctgtaa gattgtccag 720
   tgcaaccaca cacctcacca gaattgtgca aatggaagac aaaatgtttt cttcatctgt 780
   gactcctggt ctgaaaatgt tgttatgcta ttaaagtgat ttcattctga aaaaaaaaaa 840
   aaaaaaaaaa a 851
<210> 5
   <211> 1886
   <212> DNA
   <213> homo sapiens
<400> 5
   cgcgcagccc ctccggccgc gggcgcagcg ggggcgctgg tggagctgcg aagggccagg 60
   tccggcgggc ggggcggcgg ctggcactgg ctccggactc tgcccggcca gggcggcggc 120
   tccagccggg agggcgacgt ggagcggcca cgtggagcgg cccgggggag gctggcggcg 180
   ggaggcgagg cgcgggcggc gcagcagcca ggagcgccca cggagctgga cccccagagc 240
   cgcgcggcgc cgcagcagtt ccaggaagga tgttaccttt gacgatgaca gtgttaatcc 300
   tgctgctgct ccccacgggt caggctgccc caaaggatgg agtcacaagg ccagactctg 360
   aagtgcagca tcagctcctg cccaacccct tccagccagg ccaggagcag ctcggacttc 420
   tgcagagcta cctaaaggga ctaggaagga cagaagtgca actggagcat ctgagccggg 480
   agcaggttct cctctacctc tttgccctcc atgactatga ccagagtgga cagctggatg 540
   gcctggagct gctgtccatg ttgacagctg ctctggcccc tggagctgcc aactctccta 600
   ccaccaaccc ggtgatattg atagtggaca aagtgctcga gacgcaggac ctgaatgggg 660
   atgggctcat gacccctgct gagctcatca acttcccggg agtagccctc aggcacgtgg 720
   agcccggaga gccccttgct ccatctcctc aggagccaca agctgttgga aggcagtccc 780
   tattagctaa aagcccatta agacaagaaa cacaggaagc ccctggtccc agagaagaag 840
   caaagggcca ggtagaggcc agaagggagt ctttggatcc tgtccaggag cctgggggcc 900
   aggcagaggc tgatggagat gttccagggc ccagagggga agctgagggc caggcagagg 960
   ctaaaggaga tgcccctggg cccagagggg aagctggggg ccaggcagag gctgaaggag 1020
   atgcccccgg gcccagaggg gaagctgggg gccaggcaga ggccagggag aatggagagg 1080
   aggccaagga acttccaggg gaaacactgg agtctaagaa cacccaaaat gactttgagg 1140
   tgcacattgt tcaagtggag aatgatgaga tctagatctt gaagatacag gtaccccacg 1200
   aagtctcagt gccagaacat aagccctgaa gtgggcaggg gaaatgtacg ctgggacaag 1260
   gaccatctct gtgccccctg tctggtccca gtaggtatca ggtctttctg tgcagctcag 1320
   ggagacccta agttaagggg cagattacca ataaagaact gaatgaattc atcccccggg 1380
   gccacctctc tacccgtcca gcctgcccag accctctcag aggaacgggg ttggggaccg 1440
   aaaggacagg gatgccgcct gcccagtgtt tctgggcctc acggtgctcc ggcagcagag 1500
   cgcatggtgc tagccatggc cggctgcaga ggacccagtg aggaaagctc agtctatccc 1560
   tgggccccaa accctcaccg gttccccctc acctggtgtt cagacacccc atgctctcct 1620
   gcagctcagg gcaggtgacc ccatccccag taatattaat catcactaga actttttgag 1680
   agccttgtac acatcaggca tcatgctggg cattttatat atgattttat cctcacaata 1740
   attctgtagc caagcagaat tggttccatt tgacagatga agaaattgag gcagattgcg 1800
   ttaagtgctg taccctaagg tgatatgcag ctaattaaat ggcagatttg aaaaaaaaaa 1860
   aaaaaaaaaa aaaaaaaaaa aaaaaa 1886
<210> 6
   <211> 2157
   <212> DNA
   <213> homo sapiens
<400> 6
   gctcccagcc aagaacctcg gggccgctgc gcggtgggga ggagttcccc gaaacccggc      60
   cgctaagcga ggcctcctcc tcccgcagat ccgaacggcc tgggcggggt caccccggct     120
   gggacaagaa gccgccgcct gcctgcccgg gcccggggag ggggctgggg ctggggccgg     180
   aggcggggtg tgagtgggtg tgtgcggggg gcggaggctt gatgcaatcc cgataagaaa     240
   tgctcgggtg tcttgggcac ctacccgtgg ggcccgtaag gcgctactat ataaggctgc     300
   cggcccggag ccgccgcgcc gtcagagcag gagcgctgcg tccaggatct agggccacga     360
   ccatcccaac ccggcactca cagccccgca gcgcatcccg gtcgccgccc agcctcccgc     420
   acccccatcg ccggagctgc gccgagagcc ccagggaggt gccatgcgga gcgggtgtgt     480
   ggtggtccac gtatggatcc tggccggcct ctggctggcc gtggccgggc gccccctcgc    540
   cttctcggac gcggggcccc acgtgcacta cggctggggc gaccccatcc gcctgcggca     600
   cctgtacacc tccggccccc acgggctctc cagctgcttc ctgcgcatcc gtgccgacgg     660
   cgtcgtggac tgcgcgcggg gccagagcgc gcacagtttg ctggagatca aggcagtcgc     720
   tctgcggacc gtggccatca agggcgtgca cagcgtgcgg tacctctgca tgggcgccga     780
   cggcaagatg caggggctgc ttcagtactc ggaggaagac tgtgctttcg aggaggagat     840
   ccgcccagat ggctacaatg tgtaccgatc cgagaagcac cgcctcccgg tctccctgag     900
   cagtgccaaa cagcggcagc tgtacaagaa cagaggcttt cttccactct ctcatttcct     960
   gcccatgctg cccatggtcc cagaggagcc tgaggacctc aggggccact tggaatctga    1020
   catgttctct tcgcccctgg agaccgacag catggaccca tttgggcttg tcaccggact    1080
   ggaggccgtg aggagtccca gctttgagaa gtaactgaga ccatgcccgg gcctcttcac    1140
   tgctgccagg ggctgtggta cctgcagcgt gggggacgtg cttctacaag aacagtcctg    1200
   agtccacgtt ctgtttagct ttaggaagaa acatctagaa gttgtacata ttcagagttt    1260
   tccattggca gtgccagttt ctagccaata gacttgtctg atcataacat tgtaagcctg    1320
   tagcttgccc agctgctgcc tgggccccca ttctgctccc tcgaggttgc tggacaagct    1380
   gctgcactgt ctcagttctg cttgaatacc tccatcgatg gggaactcac ttcctttgga    1440
   aaaattctta tgtcaagctg aaattctcta attttttctc atcacttccc caggagcagc    1500
   cagaagacag gcagtagttt taatttcagg aacaggtgat ccactctgta aaacagcagg    1560
   taaatttcac tcaaccccat gtgggaattg atctatatct ctacttccag ggaccatttg    1620
   cccttcccaa atccctccag gccagaactg actggagcag gcatggccca ccaggcttca    1680
   ggagtagggg aagcctggag ccccactcca gccctgggac aacttgagaa ttccccctga   1740
   ggccagttct gtcatggatg ctgtcctgag aataacttgc tgtcccggtg tcacctgctt    1800
   ccatctccca gcccaccagc cctctgccca cctcacatgc ctccccatgg attggggcct    1860
   cccaggcccc ccaccttatg tcaacctgca cttcttgttc aaaaatcagg aaaagaaaag    1920
   atttgaagac cccaagtctt gtcaataact tgctgtgtgg aagcagcggg ggaagaccta    1980
   gaaccctttc cccagcactt ggttttccaa catgatattt atgagtaatt tattttgata    2040
   tgtacatctc ttattttctt acattattta tgcccccaaa ttatatttat gtatgtaagt    2100
   gaggtttgtt ttgtatatta aaatggagtt tgtttgtaaa aaaaaaaaaa aaaaaaa 2157
<210> 7
   <211> 575
   <212> DNA
   <213> homo sapiens
<400> 7
   ggcactgctg ccatgaatgc cttcctgctc tccgcactgt gcctccttgg ggcctgggcc 60
   gccttggcag gaggggtcac cgtgcaggat ggaaatttct ccttttctct ggagtcagtg 120
   aagaagctca aagacctcca ggagccccag gagcccaggg ttgggaaact caggaacttt 180
   gcacccatcc ctggtgaacc tgtggttccc atcctctgta gcaacccgaa ctttccagaa 240
   gaactcaagc ctctctgcaa ggagcccaat gcccaggaga tacttcagag gctggaggaa 300
   atcgctgagg acccgggcac atgtgaaatc tgtgcctacg ctgcctgtac cggatgctag 360
   gggggcttgc ccactgcctg cctcccctcc gcagcaggga agctcttttc tcctgcagaa 420
   agggccaccc atgatactcc actcccagca gctcaaccta ccctggtcca gtcgggagga 480
   gcagcccggg gaggaactgg gtgactggag gcctcgcccc aacactgtcc ttccctgcca 540
   cttcaacccc cagctaataa accagattcc agagt 575
<210> 8
   <211> 878
   <212> DNA
   <213> homo sapiens
<400> 8
   cagattttca ggttgattga tgtgggacag cagccacaat gaggaactcc tatagatttc 60
   tggcatcctc tctctcagtt gtcgtttctc tcctgctaat tcctgaagat gtctgtgaaa 120
   aaattattgg aggaaatgaa gtaactcctc attcaagacc ctacatggtc ctacttagtc 180
   ttgacagaaa aaccatctgt gctggggctt tgattgcaaa agactgggtg ttgactgcag 240
   ctcactgtaa cttgaacaaa aggtcccagg tcattcttgg ggctcactca ataaccaggg 300
   aagagccaac aaaacagata atgcttgtta agaaagagtt tccctatcca tgctatgacc 360
   cagccacacg cgaaggtgac cttaaacttt tacagctgac ggaaaaagca aaaattaaca 420
   aatatgtgac tatccttcat ctacctaaaa agggggatga tgtgaaacca ggaaccatgt 480
   gccaagttgc agggtggggg aggactcaca atagtgcatc ttggtccgat actctgagag 540
   aagtcaatat caccatcata gacagaaaag tctgcaatga tcgaaatcac tataatttta 600
   accctgtgat tggaatgaat atggtttgtg ctggaagcct ccgaggtgga agagactcgt 660
   gcaatggaga ttctggaagc cctttgttgt gcgagggtgt tttccgaggg gtcacttcct 720
   ttggccttga aaataaatgc ggagaccctc gtgggcctgg tgtctatatt cttctctcaa 780
   agaaacacct caactggata attatgacta tcaagggagc agtttaaata accgtttcct 840
   ttcatttact gtggcttctt aatcttttca caaataaa 878
<210> 9
   <211> 1578
   <212> DNA
   <213> homo sapiens
<400> 9
   agtgtggtac tttgtcttga ggagatgtcc tggactcaca cggaaactta gggctacgga      60
   atgaagttct cactcccatt aggtgacagg tttttagaga agccaatcag cgtcgccgcg     120
   gtcctggttc taaagtcctc gctcacccac ccggactcat tctccccaga cgccaaggat     180
   ggtggtcatg gcgccccgaa ccctcttcct gctgctctcg ggggccctga ccctgaccga      240
   gacctgggcg ggctcccact ccatgaggta tttcagcgcc gccgtgtccc ggcccggccg     300
   cggggagccc cgcttcatcg ccatgggcta cgtggacgac acgcagttcg tgcggttcga     360
   cagcgactcg gcgtgtccga ggatggagcc gcgggcgccg tgggtggagc aggaggggcc     420
   ggagtattgg gaagaggaga cacggaacac caaggcccac gcacagactg acagaatgaa     480
   cctgcagacc ctgcgcggct actacaacca gagcgaggcc agttctcaca ccctccagtg     540
   gatgattggc tgcgacctgg ggtccgacgg acgcctcctc cgcgggtatg aacagtatgc     600
   ctacgatggc aaggattacc tcgccctgaa cgaggacctg cgctcctgga ccgcagcgga     660
   cactgcggct cagatctcca agcgcaagtg tgaggcggcc aatgtggctg aacaaaggag     720
   agcctacctg gagggcacgt gcgtggagtg gctccacaga tacctggaga acgggaagga     780
   gatgctgcag cgcgcggacc cccccaagac acacgtgacc caccaccctg tctttgacta     840
   tgaggccacc ctgaggtgct gggccctggg cttctaccct gcggagatca tactgacctg     900
   gcagcgggat ggggaggacc agacccagga cgtggagctc gtggagacca ggcctgcagg     960
   ggatggaacc ttccagaagt gggcagctgt ggtggtgcct tctggagagg agcagagata    1020
   cacgtgccat gtgcagcatg aggggctgcc ggagcccctc atgctgagat ggaagcagtc    1080
   ttccctgccc accatcccca tcatgggtat cgttgctggc ctggttgtcc ttgcagctgt    1140
   agtcactgga gctgcggtcg ctgctgtgct gtggagaaag aagagctcag attgaaaagg    1200
   agggagctac tctcaggctg caatgtgaaa cagctgccct gtgtgggact gagtggcaag    1260
   tccctttgtg acttcaagaa ccctgacttc tctttgtgca gagaccagcc cacccctgtg    1320
   cccaccatga ccctcttcct catgctgaac tgcattcctt ccccaatcac ctttcctgtt    1380
   ccagaaaagg ggctgggatg tctccgtctc tgtctcaaat ttgtggtcca ctgagctata    1440
   acttacttct gtattaaaat tagaatctga gtataaattt actttttcaa attatttcca    1500
   agagagattg atgggttaat taaaggagaa gattcctgaa atttgagaga caaaataaat    1560
   ggaagacatg agaacttt                                                  1578
<210> 10
   <211> 948
   <212> DNA
   <213> homo sapiens
<400> 10
   cacacacctt aaccctgact ttttttgctc cagtttttca gaagaagtga agtcaagatg      60
   aagaaccatt tgcttttctg gggagtcctg gcggttttta ttaaggctgt tcatgtgaaa     120
   gcccaagaag atgaaaggat tgttcttgtt gacaacaaat gtaagtgtgc ccggattact     180
   tccaggatca tccgttcttc cgaagatcct aatgaggaca ttgtggagag aaacatccga     240
   attattgttc ctctgaacaa cagggagaat atctctgatc ccacctcacc attgagaacc     300
   agatttgtgt accatttgtc tgacctctgt aaaaaatgtg atcctacaga agtggagctg     360
   gataatcaga tagttactgc tacccagagc aatatctgtg atgaagacag tgctacagag     420
   acctgctaca cttatgacag aaacaagtgc tacacagctg tggtcccact cgtatatggt     480
   ggtgagacca aaatggtgga aacagcctta accccagatg cctgctatcc tgactaattt     540
   aagtcattgc tgactgcata gctctttttc ttgagaggct ctccattttg attcagaaag     600
   ttagcatatt tattaccaat gaatttgaaa ccagggcttt tttttttttt ttgggtgatg     660
   taaaaccaac tccctgccac caaaataatt aaaatagtca cattgttatc tttattaggt     720
   aatcacttct taattatatg ttcatactct aagtatcaaa atcttccaat tatcatgctc     780
   acctgaaaga ggtatgctct cttaggaata cagtttctag cattaaacaa ataaacaagg     840
   ggagaaaata aaactcaagg agtgaaaatc aggaggtgta ataaaatgtt cctcgcattc     900
   aaaaaaaaaa aaaaaaaaaa aaaaaaaaaa aaaaaaaaaa aaaaaaaa                  948
<210> 11
   <211> 947
   <212> DNA
   <213> homo sapiens
<400> 11
   ccatccctga gatcttttta taaaaaaccc agtctttgct gaccagacaa agcataccag 60
   atctcaccag agagtcctag gggactacag aaggaaaaag acaagaggca gtaggatatc 120
   tgtgtgtcct cccgctgacc acacttcctt tagtgacccg attgcctcct caagtcgcag 180
   acactatgct gcctcccatg gccctgccca gtgtgtcctg gatgctgctt tcctgcctca 240
   ttctcctgtg tcaggttcaa ggtgaagaaa cccagaagga actgccctct ccacggatca 300
   gctgtcccaa aggctccaag gcctatggct ccccctgcta tgccttgttt ttgtcaccaa 360
   aatcctggat ggatgcagat ctggcttgcc agaagcggcc ctctggaaaa ctggtgtctg 420
   tgctcagtgg ggctgaggga tccttcgtgt cctccctggt gaggagcatt agtaacagct 480
   attcatacat ctggattggg ctccatgacc ccacacaggg ctctgagcct gatggagatg 540
   gatgggagtg gagtagcact gatgtgatga attactttgc atgggagaaa aatccctcca 600
   ccatcttaaa ccctggccac tgtgggagcc tgtcaagaag cacaggattt ctgaagtgga 660
   aagattataa ctgtgatgca aagttaccct atgtctgcaa gttcaaggac tagggcaggt 720
   gggaagtcag cagcctgagc ttggcgtgca gctcatcatg gacatgagac cagtgtgaag 780
   actcaccctg gaagagaata ttctccccaa actgccctac ctgactacct tgtcatgatc 840
   ctccttcttt ttcctttttc ttcaccttca tttcaggctt ttctctgtct tccatgtctt 900
   gagatctcag agaataataa taaaaatgtt actttatacg taaaaaa 947
<210> 12
   <211> 847
   <212> DNA
   <213> homo sapiens
<400> 12
   ccatccctga gatcttttta taaaaaaccc agtctttgct gaccagacaa agcataccag 60
   atctcaccag agagtcgcag acactatgct gcctcccatg gccctgccca gtgtgtcctg 120
   gatgctgctt tcctgcctca ttctcctgtg tcaggttcaa ggtgaagaaa cccagaagga 180
   actgccctct ccacggatca gctgtcccaa aggctccaag gcctatggct ccccctgcta 240
   tgccttgttt ttgtcaccaa aatcctggat ggatgcagat ctggcttgcc agaagcggcc 300
   ctctggaaaa ctggtgtctg tgctcagtgg ggctgaggga tccttcgtgt cctccctggt 360
   gaggagcatt agtaacagct attcatacat ctggattggg ctccatgacc ccacacaggg 420
   ctctgagcct gatggagatg gatgggagtg gagtagcact gatgtgatga attactttgc 480
   atgggagaaa aatccctcca ccatcttaaa ccctggccac tgtgggagcc tgtcaagaag 540
   cacaggattt ctgaagtgga aagattataa ctgtgatgca aagttaccct atgtctgcaa 600
   gttcaaggac tagggcaggt gggaagtcag cagcctgagc ttggcgtgca gctcatcatg 660
   gacatgagac cagtgtgaag actcaccctg gaagagaata ttctccccaa actgccctac 720
   ctgactacct tgtcatgatc ctccttcttt ttcctttttc ttcaccttca tttcaggctt 780
   ttctctgtct tccatgtctt gagatctcag agaataataa taaaaatgtt actttatact 840
   taaaaaa 847
<210> 13
   <211> 1069
   <212> DNA
   <213> homo sapiens
<400> 13
   gcccctggag gaactgaacc cactatcggt catggggccg agactaaatg tggcgggttg     60
   tctttaatct gctgccaaga ggaaactcat tcaggcaagt tcagcccttt atgaggaatt    120
   cccctgtggt cacattccaa ttcctggacc tgctgccacc ctcagaactg catgctcctt    180
   cttcagactt tctaagaatg actcaggtca ttggtggagt gaagtcaaga tttccaactc    240
   agtcacctga agagatggag ataccattca tggagctgga ggtccctgga gatttgggaa    300
   ttcagataac aagctaagat aaggagtttg cctacctctg tcctagagcg aagcctgagc    360
   cttgggcgcg cagcacacca caagtatctg ttactgtgtt ttgcagaagc ttcaggcggg    420
   gatataagcc ccacaaggaa agcgctgagc agaggaggcc tcagcttgac ctgcggcagt    480
   gcagcccttg ggacttccct cgccttccac ctcctgctcg tctgcttcac aagctatcgc    540
   tatggtgttc gtgcgcaggc cgtggcccgc cttgaccaca gtgcttctgg ccctgctcgt    600
   ctgcctaggg gcgctggtcg acgcctaccc catcaaaccc gaggctcccc gcgaagacgc    660
   ctcgccggag gagctgaacc gctactacgc ctccctgcgc cactacctca acctggtcac    720
   ccggcagcgg tatgggaaaa gagacggccc ggacacgctt ctttccaaaa cgttcttccc    780
   cgacggcgag gaccgccccg tcaggtcgcg gtcggagggc ccagacctgt ggtgaggacc    840
   cctgaggcct cctgggagat ctgccaacca cgcccacgtc atttgcatac gcactcccga    900
   ccccagaaac ccggattctg cctcccgacg gcggcgtctg ggcagggttc gggtgcggcc    960
   ctccgcccgc gtctcggtgc ccccgccccc tgggctggag ggctgtgtgt ggtccttccc   1020
   tggtcccaaa ataaagagca aattccacag aaaaaaaaaa aaaaaaaaa               1069
<210> 14
   <211> 808
   <212> DNA
   <213> homo sapiens
<400> 14
   gatataaagc tcctacagct acctggcctg agaagccaac tcagactcag ccaacagaga    60
   ttgttgattt gcctcttaag caagagattc attgcagctc agcatggctc agaccagctc   120
   atacttcatg ctgatctcct gcctgatgtt tctgtctcag agccaaggcc aagaggccca   180
   gacagagttg ccccaggccc ggatcagctg cccagaaggc accaatgcct atcgctccta   240
   ctgctactac tttaatgaag accgtgagac ctgggttgat gcagatctct attgccagaa   300
   catgaattcg ggcaacctgg tgtctgtgct cacccaggcc gagggtgcct ttgtggcctc   360
   actgattaag gagagtggca ctgatgactt caatgtctgg attggcctcc atgaccccaa   420
   aaagaaccgc cgctggcact ggagcagtgg gtccctggtc tcctacaagt cctggggcat   480
   tggagcccca agcagtgtta atcctggcta ctgtgtgagc ctgacctcaa gcacaggatt   540
   ccagaaatgg aaggatgtgc cttgtgaaga caagttctcc tttgtctgca agttcaaaaa   600
   ctagaggcaa ctggaaaata catgtctaga actgatccag caattacaac ggagtcaaaa   660
   attaaaccgg accatctctc caactcaact caacctggac actctcttct ctgctgagtt   720
   tgccttgtta atcttcaata gttttaccta ccccagtctt tggaacccta aataataaaa   780
   ataaacatgt ttccactatt gtgctgtc                                      808
<210> 15
   <211> 807
   <212> DNA
   <213> homo sapiens
<400> 15
   aaaccatacc atatcccacc agagagtgac tcctgattgc ctcctcaagt cgcagacact    60
   atgctgcctc ccatggccct gcccagtgta tcttggatgc tgctttcctg cctcatgctg   120
   ctgtctcagg ttcaaggtga agaaccccag agggaactgc cctctgcacg gatccgctgt   180
   cccaaaggct ccaaggccta tggctcccac tgctatgcct tgtttttgtc accaaaatcc   240
   tggacagatg cagatctggc ctgccagaag cggccctctg gaaacctggt gtctgtgctc   300
   agtggggctg agggatcctt cgtgtcctcc ctggtgaaga gcattggtaa cagctactca   360
   tacgtctgga ttgggctcca tgaccccaca cagggcaccg agcccaatgg agaaggttgg   420
   gagtggagta gcagtgatgt gatgaattac tttgcatggg agagaaatcc ctccaccatc   480
   tcaagccccg gccactgtgc gagcctgtcg agaagcacag catttctgag gtggaaagat   540
   tataactgta atgtgaggtt accctatgtc tgcaagttca ctgactagtg caggagggaa   600
   gtcagcagcc tgtgtttggt gtgcaactca tcatgggcat gagaccagtg tgaggactca   660
   ccctggaaga gaatattcgc ttaattcccc caacctgacc acctcattct tatctttctt   720
   ctgtttcttc ctccccgctg tcatttcagt ctcttcattt tgtcatacgg cctaaggctt   780
   taaagagcaa taaaattttt agtctgc                                       807
<210> 16
   <211> 784
   <212> DNA
   <213> homo sapiens
<400> 16
   aaaccatacc atatcccacc agagagtcgc agacactatg ctgcctccca tggccctgcc    60
   cagtgtatct tggatgctgc tttcctgcct catgctgctg tctcaggttc aaggtgaaga   120
   accccagagg gaactgccct ctgcacggat ccgctgtccc aaaggctcca aggcctatgg   180
   ctcccactgc tatgccttgt ttttgtcacc aaaatcctgg acagatgcag atctggcctg   240
   ccagaagcgg ccctctggaa acctggtgtc tgtgctcagt ggggctgagg gatccttcgt   300
   gtcctccctg gtgaagagca ttggtaacag ctactcatac gtctggattg ggctccatga   360
   ccccacacag ggcaccgagc ccaatggaga aggttgggag tggagtagca gtgatgtgat   420
   gaattacttt gcatgggaga gaaatccctc caccatctca agccccggcc actgtgcgag   480
   cctgtcgaga agcacagcat ttctgaggtg gaaagattat aactgtaatg tgaggttacc   540
   ctatgtctgc aagttcactg actagtgcag gagggaagtc agcagcctgt gtttggtgtg   600
   caactcatca tgggcatgag accagtgtga ggactcaccc tggaagagaa tattcgctta   660
   attcccccaa cctgaccacc tcattcttat ctttcttctg tttcttcctc cccgctgtca   720
   tttcagtctc ttcattttgt catacggcct aaggctttaa agagcaataa aatttttagt   780
   ctgc                                                                784
<210> 17
   <211> 1002
   <212> DNA
   <213> homo sapiens
<400> 17
   gggagggtcc cttcctcagg gagcacagga actctgagac tcagcaaggg tgtcctggga    60
   gggctcgggg atgggagagt acacagattc acaactcatt cagaactgta gaagatgatg   120
   gatgtgacca agatcacttt agtcctaggg gactagagaa ggaaaatgac atgaggcagt   180
   ggggtatctg tgtgttctcc cactgaccac gctttcttta gtgactcctg attgcctcct   240
   caagtcgcag acactatgct gcctcccatg gccctgccca gtgtatcttg gatgctgctt   300
   tcctgcctca tgctgctgtc tcaggttcaa ggtgaagaac cccagaggga actgccctct   360
   gcacggatcc gctgtcccaa aggctccaag gcctatggct cccactgcta tgccttgttt   420
   ttgtcaccaa aatcctggac agatgcagat ctggcctgcc agaagcggcc ctctggaaac   480
   ctggtgtctg tgctcagtgg ggctgaggga tccttcgtgt cctccctggt gaagagcatt   540
   ggtaacagct actcatacgt ctggattggg ctccatgacc ccacacaggg caccgagccc 600
   aatggagaag gttgggagtg gagtagcagt gatgtgatga attactttgc atgggagaga 660
   aatccctcca ccatctcaag ccccggccac tgtgcgagcc tgtcgagaag cacagcattt 720
   ctgaggtgga aagattataa ctgtaatgtg aggttaccct atgtctgcaa gttcactgac 780
   tagtgcagga gggaagtcag cagcctgtgt ttggtgtgca actcatcatg ggcatgagac 840
   cagtgtgagg actcaccctg gaagagaata ttcgcttaat tcccccaacc tgaccacctc 900
   attcttatct ttcttctgtt tcttcctccc cgctgtcatt tcagtctctt cattttgtca 960
   tacggcctaa ggctttaaag agcaataaaa tttttagtct gc 1002
<210> 18
   <211> 1285
   <212> DNA
   <213> homo sapiens
<400> 18
   ataagacttt tatggatgga ttgtttttct caaataatat tatcgctttg tgactaaagt 60
   aaagattatt aattcctgag gcaagaagat ataaaagctc cagaaacgtt gactgggacc 120
   actggagaca ctgaagaagg caggggccct tagagtcttg gttgccaaac agatttgcag 180
   atcaaggaga acccaggagt ttcaaagaag cgctagtaag gtctctgaga tccttgcact 240
   agctacatcc tcagggtagg aggaagatgg cttccagaag catgcggctg ctcctattgc 300
   tgagctgcct ggccaaaaca ggagtcctgg gtgatatcat catgagaccc agctgtgctc 360
   ctggatggtt ttaccacaag tccaattgct atggttactt caggaagctg aggaactggt 420
   ctgatgccga gctcgagtgt cagtcttacg gaaacggagc ccacctggca tctatcctga 480
   gtttaaagga agccagcacc atagcagagt acataagtgg ctatcagaga agccagccga 540
   tatggattgg cctgcacgac ccacagaaga ggcagcagtg gcagtggatt gatggggcca 600
   tgtatctgta cagatcctgg tctggcaagt ccatgggtgg gaacaagcac tgtgctgaga 660
   tgagctccaa taacaacttt ttaacttgga gcagcaacga atgcaacaag cgccaacact 720
   tcctgtgcaa gtaccgacca tagagcaaga atcaagattc tgctaactcc tgcacagccc 780
   cgtcctcttc ctttctgcta gcctggctaa atctgctcat tatttcagag gggaaaccta 840
   gcaaactaag agtgataagg gccctactac actggctttt ttaggcttag agacagaaac 900
   tttagcattg gcccagtagt ggcttctagc tctaaatgtt tgccccgcca tccctttcca 960
   cagtatcctt cttccctcct cccctgtctc tggctgtctc gagcagtcta gaagagtgca 1020
   tctccagcct atgaaacagc tgggtctttg gccataagaa gtaaagattt gaagacagaa 1080
   ggaagaaact caggagtaag cttctagacc ccttcagctt ctacaccctt ctgccctctc 1140
   tccattgcct gcaccccacc ccagccactc aactcctgct tgtttttcct ttggccatag 1200
   gaaggtttac cagtagaatc cttgctaggt tgatgtgggc catacattcc tttaataaac 1260
   cattgtgtac ataagaaaaa aaaaa 1285
<210> 19
   <211> 224
   <212> PRT
   <213> homo sapiens
<400> 19
   Met Arg Val Ser Gly Val Leu Arg Leu Leu Ala Leu Ile Phe Ala Ile
   1               5                   10                  15
   Val Thr Thr Trp Met Phe Ile Arg Ser Tyr Met Ser Phe Ser Met Lys
               20                  25                  30
   Thr Ile Arg Leu Pro Arg Trp Leu Ala Ala Ser Pro Thr Lys Glu Ile
           35                  40                  45
   Gln Val Lys Lys Tyr Lys Cys Gly Leu Ile Lys Pro Cys Pro Ala Asn
       50                  55                  60
   Tyr Phe Ala Phe Lys Ile Cys Ser Gly Ala Ala Asn Val Val Gly Pro
   65                  70                  75                  80
   Thr Met Cys Phe Glu Asp Arg Met Ile Met Ser Pro Val Lys Asn Asn
                   85                  90                  95
   Val Gly Arg Gly Leu Asn Ile Ala Leu Val Asn Gly Thr Thr Gly Ala
               100                 105                 110
   Val Leu Gly Gln Lys Ala Phe Asp Met Tyr Ser Gly Asp Val Met His
           115                 120                 125
   Leu Val Lys Phe Leu Lys Glu Ile Pro Gly Gly Ala Leu Val Leu Val
       130                 135                 140
   Ala Ser Tyr Asp Asp Pro Gly Thr Lys Met Asn Asp Glu Ser Arg Lys
   145                 150                 155                 160
   Leu Phe Ser Asp Leu Gly Ser Ser Tyr Ala Lys Gln Leu Gly Phe Arg
                   165                 170                 175
   Asp Ser Trp Val Phe Ile Gly Ala Lys Asp Leu Arg Gly Lys Ser Pro
               180                 185                 190
   Phe Glu Gln Phe Leu Lys Asn Ser Pro Asp Thr Asn Lys Tyr Glu Gly
           195                 200                 205
   Trp Pro Glu Leu Leu Glu Met Glu Gly Cys Met Pro Pro Lys Pro Phe
       210                 215                 220
<210> 20
   <211> 396
   <212> PRT
   <213> homo sapiens
<400> 20
   Met Phe Leu Lys Ala Val Val Leu Thr Leu Ala Leu Val Ala Val Ala
   1 5 10 15
   Gly Ala Arg Ala Glu Val Ser Ala Asp Gln Val Ala Thr Val Met Trp
   20 25 30
   Asp Tyr Phe Ser Gln Leu Ser Asn Asn Ala Lys Glu Ala Val Glu His
   35 40 45
   Leu Gln Lys Ser Glu Leu Thr Gln Gln Leu Asn Ala Leu Phe Gln Asp
   50 55 60
   Lys Leu Gly Glu Val Asn Thr Tyr Ala Gly Asp Leu Gln Lys Lys Leu
   65 70 75 80
   Val Pro Phe Ala Thr Glu Leu His Glu Arg Leu Ala Lys Asp Ser Glu
   85 90 95
   Lys Leu Lys Glu Glu Ile Gly Lys Glu Leu Glu Glu Leu Arg Ala Arg
   100 105 110
   Leu Leu Pro His Ala Asn Glu Val Ser Gln Lys Ile Gly Asp Asn Leu
   115 120 125
   Arg Glu Leu Gln Gln Arg Leu Glu Pro Tyr Ala Asp Gln Leu Arg Thr
   130 135 140
   Gln Val Ser Thr Gln Ala Glu Gln Leu Arg Arg Gln Leu Thr Pro Tyr
   145 150 155 160
   Ala Gln Arg Met Glu Arg Val Leu Arg Glu Asn Ala Asp Ser Leu Gln
   165 170 175
   Ala Ser Leu Arg Pro His Ala Asp Glu Leu Lys Ala Lys Ile Asp Gln
   180 185 190
   Asn Val Glu Glu Leu Lys Gly Arg Leu Thr Pro Tyr Ala Asp Glu Phe
   195 200 205
   Lys Val Lys Ile Asp Gln Thr Val Glu Glu Leu Arg Arg Ser Leu Ala
   210 215 220
   Pro Tyr Ala Gln Asp Thr Gln Glu Lys Leu Asn His Gln Leu Glu Gly
   225 230 235 240
   Leu Thr Phe Gln Met Lys Lys Asn Ala Glu Glu Leu Lys Ala Arg Ile
   245 250 255
   Ser Ala Ser Ala Glu Glu Leu Arg Gln Arg Leu Ala Pro Leu Ala Glu
   260 265 270
   Asp Val Arg Gly Asn Leu Arg Gly Asn Thr Glu Gly Leu Gln Lys Ser
   275 280 285
   Leu Ala Glu Leu Gly Gly His Leu Asp Gln Gln Val Glu Glu Phe Arg
   290 295 300
   Arg Arg Val Glu Pro Tyr Gly Glu Asn Phe Asn Lys Ala Leu Val Gln
   305 310 315 320
   Gln Met Glu Gln Leu Arg Gln Lys Leu Gly Pro His Ala Gly Asp Val
   325 330 335
   Glu Gly His Leu Ser Phe Leu Glu Lys Asp Leu Arg Asp Lys Val Asn
   340 345 350
   Ser Phe Phe Ser Thr Phe Lys Glu Lys Glu Ser Gln Asp Lys Thr Leu
   355 360 365
   Ser Leu Pro Glu Leu Glu Gln Gln Gln Glu Gln Gln Gln Glu Gln Gln
   370 375 380
   Gln Glu Gln Val Gln Met Leu Ala Pro Leu Glu Ser
   385 390 395
<210> 21
   <211> 101
   <212> PRT
   <213> homo sapiens
<400> 21
   Met Gly Thr Arg Leu Leu Pro Ala Leu Phe Leu Val Leu Leu Val Leu
   1 5 10 15
   Gly Phe Glu Val Gln Gly Thr Gln Gln Pro Gln Gln Asp Glu Met Pro
   20 25 30
   Ser Pro Thr Phe Leu Thr Gln Val Lys Glu Ser Leu Ser Ser Tyr Trp
   35 40 45
   Glu Ser Ala Lys Thr Ala Ala Gln Asn Leu Tyr Glu Lys Thr Tyr Leu
   50 55 60
   Pro Ala Val Asp Glu Lys Leu Arg Asp Leu Tyr Ser Lys Ser Thr Ala
   65 70 75 80
   Ala Met Ser Thr Tyr Thr Gly Ile Phe Thr Asp Gln Val Leu Ser Val
   85 90 95
   Leu Lys Gly Glu Glu
   100
<210> 22
   <211> 115
   <212> PRT
   <213> homo sapiens
<400> 22
   Met Asn Ser Gly Val Cys Leu Cys Val Leu Met Ala Val Leu Ala Ala
   1 5 10 15
   Gly Ala Leu Thr Gln Pro Val Pro Pro Ala Asp Pro Ala Gly Ser Gly
   20 25 30
   Leu Gln Arg Ala Glu Glu Ala Pro Arg Arg Gln Leu Arg Val Ser Gln
   35 40 45
   Arg Thr Asp Gly Glu Ser Arg Ala His Leu Gly Ala Leu Leu Ala Arg
   50 55 60
   Tyr Ile Gln Gln Ala Arg Lys Ala Pro Ser Gly Arg Met Ser Ile Val
   65 70 75 80
   Lys Asn Leu Gln Asn Leu Asp Pro Ser His Arg Ile Ser Asp Arg Asp
   85 90 95
   Tyr Met Gly Trp Met Asp Phe Gly Arg Arg Ser Ala Glu Glu Tyr Glu
   100 105 110
   Tyr Pro Ser
   115
<210> 23
   <211> 301
   <212> PRT
   <213> homo sapiens
<400> 23
   Met Leu Pro Leu Thr Met Thr Val Leu Ile Leu Leu Leu Leu Pro Thr
   1 5 10 15
   Gly Gln Ala Ala Pro Lys Asp Gly Val Thr Arg Pro Asp Ser Glu Val
   20 25 30
   Gln His Gln Leu Leu Pro Asn Pro Phe Gln Pro Gly Gln Glu Gln Leu
   35 40 45
   Gly Leu Leu Gln Ser Tyr Leu Lys Gly Leu Gly Arg Thr Glu Val Gln
   50 55 60
   Leu Glu His Leu Ser Arg Glu Gln Val Leu Leu Tyr Leu Phe Ala Leu
   65 70 75 80
   His Asp Tyr Asp Gln Ser Gly Gln Leu Asp Gly Leu Glu Leu Leu Ser
   85 90 95
   Met Leu Thr Ala Ala Leu Ala Pro Gly Ala Ala Asn Ser Pro Thr Thr
   100 105 110
   Asn Pro Val Ile Leu Ile Val Asp Lys Val Leu Glu Thr Gln Asp Leu
   115 120 125
   Asn Gly Asp Gly Leu Met Thr Pro Ala Glu Leu Ile Asn Phe Pro Gly
   130 135 140
   Val Ala Leu Arg His Val Glu Pro Gly Glu Pro Leu Ala Pro Ser Pro
   145 150 155 160
   Gln Glu Pro Gln Ala Val Gly Arg Gln Ser Leu Leu Ala Lys Ser Pro
   165 170 175
   Leu Arg Gln Glu Thr Gln Glu Ala Pro Gly Pro Arg Glu Glu Ala Lys
   180 185 190
   Gly Gln Val Glu Ala Arg Arg Glu Ser Leu Asp Pro Val Gln Glu Pro
   195 200 205
   Gly Gly Gln Ala Glu Ala Asp Gly Asp Val Pro Gly Pro Arg Gly Glu
   210 215 220
   Ala Glu Gly Gln Ala Glu Ala Lys Gly Asp Ala Pro Gly Pro Arg Gly
   225 230 235 240
   Glu Ala Gly Gly Gln Ala Glu Ala Glu Gly Asp Ala Pro Gly Pro Arg
   245 250 255
   Gly Glu Ala Gly Gly Gln Ala Glu Ala Arg Glu Asn Gly Glu Glu Ala
   260 265 270
   Lys Glu Leu Pro Gly Glu Thr Leu Glu Ser Lys Asn Thr Gln Asn Asp
   275 280 285
   Phe Glu Val His Ile Val Gln Val Glu Asn Asp Glu Ile
   290 295 300
<210> 24
   <211> 216
   <212> PRT
   <213> homo sapiens
<400> 24
   Met Arg Ser Gly Cys Val Val Val His Val Trp Ile Leu Ala Gly Leu
   1 5 10 15
   Trp Leu Ala Val Ala Gly Arg Pro Leu Ala Phe Ser Asp Ala Gly Pro
   20 25 30
   His Val His Tyr Gly Trp Gly Asp Pro Ile Arg Leu Arg His Leu Tyr
   35 40 45
   Thr Ser Gly Pro His Gly Leu Ser Ser Cys Phe Leu Arg Ile Arg Ala
       50                  55                  60
   Asp Gly Val Val Asp Cys Ala Arg Gly Gln Ser Ala His Ser Leu Leu
   65                  70                  75                  80
   Glu Ile Lys Ala Val Ala Leu Arg Thr Val Ala Ile Lys Gly Val His
                   85                  90                  95
   Ser Val Arg Tyr Leu Cys Met Gly Ala Asp Gly Lys Met Gln Gly Leu
               100                 105                 110
   Leu Gln Tyr Ser Glu Glu Asp Cys Ala Phe Glu Glu Glu Ile Arg Pro
           115                 120                 125
   Asp Gly Tyr Asn Val Tyr Arg Ser Glu Lys His Arg Leu Pro Val Ser
       130                 135                 140
   Leu Ser Ser Ala Lys Gln Arg Gln Leu Tyr Lys Asn Arg Gly Phe Leu
   145                 150                 155                 160
   Pro Leu Ser His Phe Leu Pro Met Leu Pro Met Val Pro Glu Glu Pro
                   165                 170                 175
   Glu Asp Leu Arg Gly His Leu Glu Ser Asp Met Phe Ser Ser Pro Leu
               180                 185                 190
   Glu Thr Asp Ser Met Asp Pro Phe Gly Leu Val Thr Gly Leu Glu Ala
           195                 200                 205
   Val Arg Ser Pro Ser Phe Glu Lys
       210                 215
<210> 25
   <211> 115
   <212> PRT
   <213> homo sapiens
<400> 25
   Met Asn Ala Phe Leu Leu Ser Ala Leu Cys Leu Leu Gly Ala Trp Ala
   1               5                   10                  15
   Ala Leu Ala Gly Gly Val Thr Val Gln Asp Gly Asn Phe Ser Phe Ser
               20                  25                  30
   Leu Glu Ser Val Lys Lys Leu Lys Asp Leu Gln Glu Pro Gln Glu Pro
           35                  40                  45
   Arg Val Gly Lys Leu Arg Asn Phe Ala Pro Ile Pro Gly Glu Pro Val
       50                  55                  60
   Val Pro Ile Leu Cys Ser Asn Pro Asn Phe Pro Glu Glu Leu Lys Pro
   65 70 75 80
   Leu Cys Lys Glu Pro Asn Ala Gln Glu Ile Leu Gln Arg Leu Glu Glu
   85 90 95
   Ile Ala Glu Asp Pro Gly Thr Cys Glu Ile Cys Ala Tyr Ala Ala Cys
   100 105 110
   Thr Gly Cys
   115
<210> 26
   <211> 262
   <212> PRT
   <213> homo sapiens
<400> 26
   Met Arg Asn Ser Tyr Arg Phe Leu Ala Ser Ser Leu Ser Val Val Val
   1 5 10 15
   Ser Leu Leu Leu Ile Pro Glu Asp Val Cys Glu Lys Ile Ile Gly Gly
   20 25 30
   Asn Glu Val Thr Pro His Ser Arg Pro Tyr Met Val Leu Leu Ser Leu
   35 40 45
   Asp Arg Lys Thr Ile Cys Ala Gly Ala Leu Ile Ala Lys Asp Trp Val
   50 55 60
   Leu Thr Ala Ala His Cys Asn Leu Asn Lys Arg Ser Gln Val Ile Leu
   65 70 75 80
   Gly Ala His Ser Ile Thr Arg Glu Glu Pro Thr Lys Gln Ile Met Leu
   85 90 95
   Val Lys Lys Glu Phe Pro Tyr Pro Cys Tyr Asp Pro Ala Thr Arg Glu
   100 105 110
   Gly Asp Leu Lys Leu Leu Gln Leu Thr Glu Lys Ala Lys Ile Asn Lys
   115 120 125
   Tyr Val Thr Ile Leu His Leu Pro Lys Lys Gly Asp Asp Val Lys Pro
   130 135 140
   Gly Thr Met Cys Gln Val Ala Gly Trp Gly Arg Thr His Asn Ser Ala
   145 150 155 160
   Ser Trp Ser Asp Thr Leu Arg Glu Val Asn Ile Thr Ile Ile Asp Arg
   165 170 175
   Lys Val Cys Asn Asp Arg Asn His Tyr Asn Phe Asn Pro Val Ile Gly
   180 185 190
   Met Asn Met Val Cys Ala Gly Ser Leu Arg Gly Gly Arg Asp Ser Cys
   195 200 205
   Asn Gly Asp Ser Gly Ser Pro Leu Leu Cys Glu Gly Val Phe Arg Gly
   210 215 220
   Val Thr Ser Phe Gly Leu Glu Asn Lys Cys Gly Asp Pro Arg Gly Pro
   225 230 235 240
   Gly Val Tyr Ile Leu Leu Ser Lys Lys His Leu Asn Trp Ile Ile Met
   245 250 255
   Thr Ile Lys Gly Ala Val
   260
<210> 27
   <211> 338
   <212> PRT
   <213> homo sapiens
<400> 27
   Met Val Val Met Ala Pro Arg Thr Leu Phe Leu Leu Leu Ser Gly Ala
   1 5 10 15
   Leu Thr Leu Thr Glu Thr Trp Ala Gly Ser His Ser Met Arg Tyr Phe
   20 25 30
   Ser Ala Ala Val Ser Arg Pro Gly Arg Gly Glu Pro Arg Phe Ile Ala
   35 40 45
   Met Gly Tyr Val Asp Asp Thr Gln Phe Val Arg Phe Asp Ser Asp Ser
   50 55 60
   Ala Cys Pro Arg Met Glu Pro Arg Ala Pro Trp Val Glu Gln Glu Gly
   65 70 75 80
   Pro Glu Tyr Trp Glu Glu Glu Thr Arg Asn Thr Lys Ala His Ala Gln
   85 90 95
   Thr Asp Arg Met Asn Leu Gln Thr Leu Arg Gly Tyr Tyr Asn Gln Ser
   100 105 110
   Glu Ala Ser Ser His Thr Leu Gln Trp Met Ile Gly Cys Asp Leu Gly
   115 120 125
   Ser Asp Gly Arg Leu Leu Arg Gly Tyr Glu Gln Tyr Ala Tyr Asp Gly
   130 135 140
   Lys Asp Tyr Leu Ala Leu Asn Glu Asp Leu Arg Ser Trp Thr Ala Ala
   145 150 155 160
   Asp Thr Ala Ala Gln Ile Ser Lys Arg Lys Cys Glu Ala Ala Asn Val
   165 170 175
   Ala Glu Gln Arg Arg Ala Tyr Leu Glu Gly Thr Cys Val Glu Trp Leu
   180 185 190
   His Arg Tyr Leu Glu Asn Gly Lys Glu Met Leu Gln Arg Ala Asp Pro
   195 200 205
   Pro Lys Thr His Val Thr His His Pro Val Phe Asp Tyr Glu Ala Thr
   210 215 220
   Leu Arg Cys Trp Ala Leu Gly Phe Tyr Pro Ala Glu Ile Ile Leu Thr
   225 230 235 240
   Trp Gln Arg Asp Gly Glu Asp Gln Thr Gln Asp Val Glu Leu Val Glu
   245 250 255
   Thr Arg Pro Ala Gly Asp Gly Thr Phe Gln Lys Trp Ala Ala Val Val
   260 265 270
   Val Pro Ser Gly Glu Glu Gln Arg Tyr Thr Cys His Val Gln His Glu
   275 280 285
   Gly Leu Pro Glu Pro Leu Met Leu Arg Trp Lys Gln Ser Ser Leu Pro
   290 295 300
   Thr Ile Pro Ile Met Gly Ile Val Ala Gly Leu Val Val Leu Ala Ala
   305 310 315 320
   Val Val Thr Gly Ala Ala Val Ala Ala Val Leu Trp Arg Lys Lys Ser
   325 330 335
   Ser Asp
<210> 28
   <211> 159
   <212> PRT
   <213> homo sapiens
<400> 28
   Met Lys Asn His Leu Leu Phe Trp Gly Val Leu Ala Val Phe Ile Lys
   1 5 10 15
   Ala Val His Val Lys Ala Gln Glu Asp Glu Arg Ile Val Leu Val Asp
   20 25 30
   Asn Lys Cys Lys Cys Ala Arg Ile Thr Ser Arg Ile Ile Arg Ser Ser
   35 40 45
   Glu Asp Pro Asn Glu Asp Ile Val Glu Arg Asn Ile Arg Ile Ile Val
   50 55 60
   Pro Leu Asn Asn Arg Glu Asn Ile Ser Asp Pro Thr Ser Pro Leu Arg
   65                  70                  75                  80
   Thr Arg Phe Val Tyr His Leu Ser Asp Leu Cys Lys Lys Cys Asp Pro
                   85                  90                  95
   Thr Glu Val Glu Leu Asp Asn Gln Ile Val Thr Ala Thr Gln Ser Asn
               100                 105                 110
   Ile Cys Asp Glu Asp Ser Ala Thr Glu Thr Cys Tyr Thr Tyr Asp Arg
           115                 120                 125
   Asn Lys Cys Tyr Thr Ala Val Val Pro Leu Val Tyr Gly Gly Glu Thr
       130                 135                 140
   Lys Met Val Glu Thr Ala Leu Thr Pro Asp Ala Cys Tyr Pro Asp
   145                 150                 155
<210> 29
   <211> 175
   <212> PRT
   <213> homo sapiens
<400> 29
   Met Leu Pro Pro Met Ala Leu Pro Ser Val Ser Trp Met Leu Leu Ser
   1               5                   10                  15
   Cys Leu Ile Leu Leu Cys Gln Val Gln Gly Glu Glu Thr Gln Lys Glu
               20                  25                  30
   Leu Pro Ser Pro Arg Ile Ser Cys Pro Lys Gly Ser Lys Ala Tyr Gly
           35                  40                  45
   Ser Pro Cys Tyr Ala Leu Phe Leu Ser Pro Lys Ser Trp Met Asp Ala
       50                  55                  60
   Asp Leu Ala Cys Gln Lys Arg Pro Ser Gly Lys Leu Val Ser Val Leu
   65                  70                  75                  80
   Ser Gly Ala Glu Gly Ser Phe Val Ser Ser Leu Val Arg Ser Ile Ser
                   85                  90                  95
   Asn Ser Tyr Ser Tyr Ile Trp Ile Gly Leu His Asp Pro Thr Gln Gly
               100                 105                 110
   Ser Glu Pro Asp Gly Asp Gly Trp Glu Trp Ser Ser Thr Asp Val Met
           115                 120                 125
   Asn Tyr Phe Ala Trp Glu Lys Asn Pro Ser Thr Ile Leu Asn Pro Gly
       130                 135                 140
   His Cys Gly Ser Leu Ser Arg Ser Thr Gly Phe Leu Lys Trp Lys Asp
   145 150 155 160
   Tyr Asn Cys Asp Ala Lys Leu Pro Tyr Val Cys Lys Phe Lys Asp
   165 170 175
<210> 30
   <211> 97
   <212> PRT
   <213> homo sapiens
<400> 30
   Met Val Phe Val Arg Arg Pro Trp Pro Ala Leu Thr Thr Val Leu Leu
   1 5 10 15
   Ala Leu Leu Val Cys Leu Gly Ala Leu Val Asp Ala Tyr Pro Ile Lys
   20 25 30
   Pro Glu Ala Pro Arg Glu Asp Ala Ser Pro Glu Glu Leu Asn Arg Tyr
   35 40 45
   Tyr Ala Ser Leu Arg His Tyr Leu Asn Leu Val Thr Arg Gln Arg Tyr
   50 55 60
   Gly Lys Arg Asp Gly Pro Asp Thr Leu Leu Ser Lys Thr Phe Phe Pro
   65 70 75 80
   Asp Gly Glu Asp Arg Pro Val Arg Ser Arg Ser Glu Gly Pro Asp Leu
   85 90 95
   Trp
<210> 31
   <211> 166
   <212> PRT
   <213> homo sapiens
<400> 31
   Met Ala Gln Thr Ser Ser Tyr Phe Met Leu Ile Ser Cys Leu Met Phe
   1 5 10 15
   Leu Ser Gln Ser Gln Gly Gln Glu Ala Gln Thr Glu Leu Pro Gln Ala
   20 25 30
   Arg Ile Ser Cys Pro Glu Gly Thr Asn Ala Tyr Arg Ser Tyr Cys Tyr
   35 40 45
   Tyr Phe Asn Glu Asp Arg Glu Thr Trp Val Asp Ala Asp Leu Tyr Cys
   50 55 60
   Gln Asn Met Asn Ser Gly Asn Leu Val Ser Val Leu Thr Gln Ala Glu
   65 70 75 80
   Gly Ala Phe Val Ala Ser Leu Ile Lys Glu Ser Gly Thr Asp Asp Phe
                   85                  90                  95
   Asn Val Trp Ile Gly Leu His Asp Pro Lys Lys Asn Arg Arg Trp His
               100                 105                 110
   Trp Ser Ser Gly Ser Leu Val Ser Tyr Lys Ser Trp Gly Ile Gly Ala
           115                 120                 125
   Pro Ser Ser Val Asn Pro Gly Tyr Cys Val Ser Leu Thr Ser Ser Thr
       130                 135                 140
   Gly Phe Gln Lys Trp Lys Asp Val Pro Cys Glu Asp Lys Phe Ser Phe
   145                 150                 155                 160
   Val Cys Lys Phe Lys Asn
                   165
<210> 32
   <211> 175
   <212> PRT
   <213> homo sapiens
<400> 32
   Met Leu Pro Pro Met Ala Leu Pro Ser Val Ser Trp Met Leu Leu Ser
   1               5                   10                  15
   Cys Leu Met Leu Leu Ser Gln Val Gln Gly Glu Glu Pro Gln Arg Glu
               20                  25                  30
   Leu Pro Ser Ala Arg Ile Arg Cys Pro Lys Gly Ser Lys Ala Tyr Gly
           35                  40                  45
   Ser His Cys Tyr Ala Leu Phe Leu Ser Pro Lys Ser Trp Thr Asp Ala
       50                  55                  60
   Asp Leu Ala Cys Gln Lys Arg Pro Ser Gly Asn Leu Val Ser Val Leu
   65                  70                  75                  80
   Ser Gly Ala Glu Gly Ser Phe Val Ser Ser Leu Val Lys Ser Ile Gly
                   85                  90                  95
   Asn Ser Tyr Ser Tyr Val Trp Ile Gly Leu His Asp Pro Thr Gln Gly
               100                 105                 110
   Thr Glu Pro Asn Gly Glu Gly Trp Glu Trp Ser Ser Ser Asp Val Met
           115                 120                 125
   Asn Tyr Phe Ala Trp Glu Arg Asn Pro Ser Thr Ile Ser Ser Pro Gly
       130                 135                 140
   His Cys Ala Ser Leu Ser Arg Ser Thr Ala Phe Leu Arg Trp Lys Asp
   145                 150                 155                 160
   Tyr Asn Cys Asn Val Arg Leu Pro Tyr Val Cys Lys Phe Thr Asp
                   165                 170                 175
<210> 33
   <211> 158
   <212> PRT
   <213> homo sapiens
<400> 33
   Met Ala Ser Arg Ser Met Arg Leu Leu Leu Leu Leu Ser Cys Leu Ala
   1               5                   10                  15
   Lys Thr Gly Val Leu Gly Asp Ile Ile Met Arg Pro Ser Cys Ala Pro
               20                  25                  30
   Gly Trp Phe Tyr His Lys Ser Asn Cys Tyr Gly Tyr Phe Arg Lys Leu
           35                  40                  45
   Arg Asn Trp Ser Asp Ala Glu Leu Glu Cys Gln Ser Tyr Gly Asn Gly
       50                  55                  60
   Ala His Leu Ala Ser Ile Leu Ser Leu Lys Glu Ala Ser Thr Ile Ala
   65                  70                  75                  80
   Glu Tyr Ile Ser Gly Tyr Gln Arg Ser Gln Pro Ile Trp Ile Gly Leu
                   85                  90                  95
   His Asp Pro Gln Lys Arg Gln Gln Trp Gln Trp Ile Asp Gly Ala Met
               100                 105                 110
   Tyr Leu Tyr Arg Ser Trp Ser Gly Lys Ser Met Gly Gly Asn Lys His
           115                 120                 125
   Cys Ala Glu Met Ser Ser Asn Asn Asn Phe Leu Thr Trp Ser Ser Asn
       130                 135                 140
   Glu Cys Asn Lys Arg Gln His Phe Leu Cys Lys Tyr Arg Pro
   145                 150                 155
<210> 34
   <211> 20
   <212> DNA
   <213> artificial
<220>
   <223> primer
<400> 34
   ctgcccagcc aactactttg 20
<210> 35
   <211> 19
   <212> DNA
   <213> artificial
<220>
   <223> primer
<400> 35
   ctcccgtggt tccattcac 19
<210> 36
   <211> 21
   <212> DNA
   <213> artificial
<220>
   <223> primer
<400> 36
   ccaagatcga ccagaacgtg g 21
<210> 37
   <211> 20
   <212> DNA
   <213> artificial
<220>
   <223> primer
<400> 37
   gtcctgagca tagggagcca 20
<210> 38
   <211> 20
   <212> DNA
   <213> artificial
<220>
   <223> primer
<400> 38
   cccagagcaa aaagcgactc 20
<210> 39
   <211> 24
   <212> DNA
   <213> artificial
<220>
   <223> primer
<400> 39
   accatccatg cctacaacaa aagg 24
<210> 40
   <211> 21
   <212> DNA
   <213> artificial
<220>
   <223> primer
<400> 40
   caacaggctg aaggctacga t 21
<210> 41
   <211> 21
   <212> DNA
   <213> artificial
<220>
   <223> primer
<400> 41
   cgatttttgc ggagaccttg g 21
<210> 42
   <211> 21
   <212> DNA
   <213> artificial
<220>
   <223> primer
<400> 42
   cagaagtgcg aagaggaggt c 21
<210> 43
   <211> 22
   <212> DNA
   <213> artificial
<220>
   <223> primer
<400> 43
   tcatcttaga ggccacgaac at 22
<210> 44
   <211> 18
   <212> DNA
   <213> artificial
<220>
   <223> primer
<400> 44
   tccagccaat gcctttgc 18
<210> 45
   <211> 24
   <212> DNA
   <213> artificial
<220>
   <223> primer
<400> 45
   tggagattac ttttcagtgc agaa 24
<210> 46
   <211> 22
   <212> DNA
   <213> artificial
<220>
   <223> primer
<400> 46
   tagagatcga cgggatgaaa ct 22
<210> 47
   <211> 19
   <212> DNA
   <213> artificial
<220>
   <223> primer
<400> 47
   cagatccgcc actccttct 19
<210> 48
   <211> 21
   <212> DNA
   <213> artificial
<220>
   <223> primer
<400> 48
   tggtggatgg gaagctgtct a 21
<210> 49
   <211> 21
   <212> DNA
   <213> artificial
<220>
   <223> primer
<400> 49
   ttcttgcggt aggctgcata g 21
<210> 50
   <211> 22
   <212> DNA
   <213> artificial
<220>
   <223> primer
<400> 50
   gactcttgcg tcaacttcaa gg 22
<210> 51
   <211> 21
   <212> DNA
   <213> artificial
<220>
   <223> primer
<400> 51
   caggctgtct tttgtcaacg a 21
<210> 52
   <211> 22
   <212> DNA
   <213> artificial
<220>
   <223> primer
<400> 52
   atacaagctc acgtactcca ct 22
<210> 53
   <211> 21
   <212> DNA
   <213> artificial
<220>
   <223> primer
<400> 53
   tccacagtaa cacaacgtcc a 21
<210> 54
   <211> 22
   <212> DNA
   <213> artificial
<220>
   <223> primer
<400> 54
   tttggagtgg tagtaaaaag gg 22
<210> 55
   <211> 22
   <212> DNA
   <213> artificial
<220>
   <223> primer
<400> 55
   tgacatcagg gactcagagt ag 22
<210> 56
   <211> 19
   <212> DNA
   <213> artificial
<220>
   <223> primer
<400> 56
   ccggagaccc ttagatcga 19
<210> 57
   <211> 26
   <212> DNA
   <213> artificial
<220>
   <223> primer
<400> 57
   tagcctgtaa aagatttctg caaacc 26
<210> 58
   <211> 23
   <212> DNA
   <213> artificial
<220>
   <223> primer
<400> 58
   ccgaaaccca tcagtccgtt taa 23
<210> 59
   <211> 22
   <212> DNA
   <213> artificial
<220>
   <223> primer
<400> 59
   acataggtgg ctgtccagac aa 22